# EUROPEAN PATENT APPLICATION

(11) **EP 3 901 777 A1**
(43) Date of publication of application: **27.10.2021**
(21) Application number: 19918478.9
(22) Date of filing: 06.03.2019
(51) Int. Cl.: G06F 13/00

(54) **INFORMATION PROVISION METHOD, PROGRAM, INFORMATION PROCESSING DEVICE, BATTERY, TERMINAL DEVICE, AND INFORMATION PROVISION SYSTEM**

(71) Applicant: Japan Tobacco Inc., Tokyo 105-6927 (JP)
(72) Inventor: HASHIMOTO, Kentaro, Tokyo 105-6927 (JP)
(74) Representative: Hoffmann Eitle
(86) International application number: PCT/JP2019/008954
(87) International publication number: WO 2020/179031

(57) **Abstract**

An information provision method comprising: a step for acquiring information, relating to a beacon signal which is received by a user terminal associated with an aerosol generation device, from a server which is included in a message exchange system capable of transmitting a message to the user terminal; a step for, on the basis of the acquired information relating to the beacon signal, creating provision information which pertains to the aerosol generation device and which is provided to a user of the aerosol generation device; and a step for transmitting the provision information to the server.

## Description

### Technical Field

The present invention relates to an information provision method, a program, an information processing device, a battery, a terminal device, and an information provision system.

### Background Art

Regarding systems providing services by using beacon signals, a technique for distributing content tailored to users has been known (see, for example, PTL 1). In this technique, when a user terminal receives a beacon signal, the user terminal transmits information indicating reception of the beacon signal to a server. The user terminal receives predetermined information based on the information which is transmitted to the server and which indicates reception of the beacon signal. The predetermined information includes at least information associated in advance with the beacon signal.

### Citation List

### Patent Literature

PTL 1: Japanese Unexamined Patent Application Publication No. 2017-204102

### Summary of Invention

### Technical Problem

In the technique described above, the predetermined information that the user terminal receives is associated in advance with the beacon signal It is thus difficult to increase the variety of information to be received by the user terminal.

The present invention has been made in view of the above-described points, and an object of the present invention is to provide an information provision method, a program, an information processing device, a battery, a terminal device, and an information provision system that are capable of increasing the variety of information to be provided to a user terminal that receives a beacon signal in a system providing a service by using a beacon signal.

### Solution to Problem

According to an embodiment of the present invention, there is provided an information provision method including a step for acquiring information, relating to a beacon signal which is received by a user terminal associated with an aerosol generation device, from a server which is included in a message exchange system capable of transmitting a message to the user terminal; a step for, on the basis of the acquired information relating to the beacon signal, creating provision information which pertains to the aerosol generation device and which is provided to a user of the aerosol generation device; and a step for transmitting the provision information to the server.

According to an embodiment, the information provision method may further include a step for, on the basis of the information relating to the beacon signal, deriving information indicating an acquisition status of the information relating to the beacon signal; and a step for, on the basis of the information indicating the acquisition status, creating the provision information.

According to an embodiment, the acquisition status may be the number of times that the user terminal has received the beacon signal during a predetermined period of time.

According to an embodiment, the acquisition status may be a time during which the user terminal continues to receive the beacon signal.

According to an embodiment, the information provision method may further include a step for, on the basis of the information indicating the acquisition status, creating reward information, which indicates a reward to be granted to the user of the aerosol generation device, as the provision information; and a step for transmitting the reward information to the server.

According to an embodiment, the information provision method may further include a step for transmitting the provision information to the server at a timing determined on the basis of the information indicating the acquisition status.

According to an embodiment, the beacon signal may be transmitted from a predetermined device provided in a place where the aerosol generation device is able to be used.

According to an embodiment, the information provision method may further include a step for acquiring the information, relating to the beacon signal which is transmitted on the basis of detection of the aerosol generation device, from the server.

According to an embodiment, the information provision method may further include a step for acquiring the information, relating to the beacon signal which is transmitted on the basis of detection of a predetermined signal transmitted from the aerosol generation device, from the server.

According to an embodiment, the information provision method may further include a step for acquiring the information, relating to the beacon signal which is transmitted from the aerosol generation device and received by another user terminal different from the user terminal, from the server.

According to an embodiment, the information provision method may further include a step for acquiring the information, relating to the beacon signal which is transmitted from the aerosol generation device in accordance with interruption of connection between the user terminal and the aerosol generation device, from the server.

According to an embodiment, the information provision method may further include a step for acquiring the information, relating to the beacon signal which includes location information of the aerosol generation device, from the server.

According to an embodiment, the information provision method may further include a step for, on the basis of the location information, acquiring information, relating to the location information of the aerosol generation device, from the server, for the user of the aerosol generation device.

According to an embodiment of the present invention, there is provided a program for causing a computer to execute a process for acquiring information, relating to a beacon signal which is received by a user terminal associated with an aerosol generation device, from a server which is included in a message exchange system capable of transmitting a message to the user terminal; a process for, on the basis of the acquired information relating to the beacon signal, creating provision information which pertains to the aerosol generation device and which is provided to a user of the aerosol generation device; and a process for transmitting the provision information to the server.

According to an embodiment of the present invention, there is provided an information processing device including an acquisition unit that acquires information, relating to a beacon signal which is received by a user terminal associated with an aerosol generation device, from a server which is included in a message exchange system capable of transmitting a message to the user terminal; a creation unit that creates, on the basis of the information relating to the beacon signal acquired by the acquisition unit, provision information which pertains to the aerosol generation device and which is provided to a user of the aerosol generation device; and a communication unit that transmits the provision information to the server.

According to an embodiment of the present invention, there is provided an information processing device including an acquisition unit that acquires information relating to a beacon signal which is received by a user terminal associated with an aerosol generation device; a creation unit that creates, on the basis of the information relating to the beacon signal acquired by the acquisition unit, provision information which pertains to the aerosol generation device and which is provided to a user of the aerosol generation device; and a communication unit that transmits the provision information to the user terminal.

According to an embodiment of the present invention, there is provided a battery included in an aerosol generation device, the battery including a communication unit that communicates with a user terminal; and a creation unit that creates a beacon signal in a case where connection with the user terminal is interrupted. The communication unit transmits the beacon signal created by the creation unit.

According to an embodiment of the present invention, there is provided a terminal device associated with an aerosol generation device, the terminal device including a communication unit that receives a beacon signal; and a creation unit that creates identification information which includes a beacon identifier that is able to uniquely identify the beacon signal which is included in the received beacon signal and a user identifier that is able to uniquely identify a user of the terminal device. The communication unit transmits the identification information to a server which is included in a message exchange system capable of transmitting a message to the terminal device.

According to an embodiment of the present invention, there is provided an information provision system including an aerosol generation device that generates an aerosol; a terminal device that is capable of communicating and connecting with the aerosol generation device; a server that is included in a message exchange system capable of transmitting a message to the terminal device; and an information processing device that receives information, relating to a beacon signal which is received by a user terminal associated with the aerosol generation device, from the server. The information processing device creates, on the basis of the acquired information relating to the beacon signal, provision information which pertains to the aerosol generation device and which is provided to a user of the aerosol generation device, and transmits the created provision information to the server.

### Advantageous Effects of Invention

According to an embodiment of the present invention, an information provision method, a program, an information processing device, a battery, a terminal device, and an information provision system that are capable of increasing the variety of information to be provided to a user terminal that receives a beacon signal in a system providing a service by using a beacon signal, can be provided.

### Brief Description of Drawings

[Fig. 1] Fig. 1 is a diagram illustrating an example of an information provision system according to a first embodiment.
[Fig. 2] Fig. 2 is a block diagram illustrating a schematic configuration of an aerosol generation device according to the first embodiment.
[Fig. 3] Fig. 3 is a block diagram illustrating another schematic configuration of an aerosol generation device according to the first embodiment.
[Fig. 4] Fig. 4 is a block diagram illustrating another schematic configuration of the aerosol generation device according to the first embodiment.
[Fig. 5] Fig. 5 is a block diagram illustrating another schematic configuration of the aerosol generation device according to the first embodiment.
[Fig. 6] Fig. 6 is a block diagram illustrating another schematic configuration of the aerosol generation device according to the first embodiment.
[Fig. 7] Fig. 7 is a block diagram illustrating another schematic configuration of the aerosol generation device according to the first embodiment.
[Fig. 8] Fig. 8 is a block diagram illustrating an example of an information provision system according to the first embodiment.
[Fig. 9] Fig. 9 is a diagram illustrating an example of beacon related information.
[Fig. 10] Fig. 10 is a diagram illustrating an example of user notification information.
[Fig. 11] Fig. 11 is a sequence chart illustrating an example of an operation of the information provision system according to the first embodiment.
[Fig. 12] Fig. 12 is a block diagram illustrating an example of an information processing device according to a modification of the first embodiment.
[Fig. 13] Fig. 13 is a diagram illustrating an example of point granting information.
[Fig. 14] Fig. 14 is a diagram illustrating an example of point information.
[Fig. 15] Fig. 15 is a block diagram illustrating an example of an aerosol generation device and a beacon transmission device included in an information provision system according to a second embodiment.
[Fig. 16] Fig. 16 is a sequence chart illustrating an example of an operation of the information provision system according to the second embodiment.
[Fig. 17] Fig. 17 is a diagram illustrating an example of an information provision system according to a third embodiment.
[Fig. 18] Fig. 18 is a block diagram illustrating an example of the information provision system according to the third embodiment.
[Fig. 19] Fig. 19 is a sequence chart illustrating an example of an operation of the information provision system according to the third embodiment.
[Fig. 20] Fig. 20 is a sequence chart illustrating an example of an operation of the information provision system according to the third embodiment.
[Fig. 21] Fig. 21 is a block diagram illustrating a schematic functional configuration of a battery device.
[Fig. 22] Fig. 22 is a block diagram illustrating another schematic functional configuration of the battery device. Description of Embodiments

Next, an information provision method, a program, an information processing device, a battery, a terminal device, and an information provision system according to embodiments will be described with reference to drawings. Embodiments described below are merely examples and embodiments to which the present invention is applied are not limited to the embodiments described below.

In all the drawings used for explaining embodiments, components having the same functions are referred to with the same signs and redundant explanation will be omitted.

Furthermore, "based on XX" in this application represents "based on at least XX" and also includes being based on another element as well as XX. Furthermore, "based on XX" is not limited to a case where XX is directly used but also includes being based on an object obtained by operation or processing on XX. "XX" is a desired element (for example, desired information).

### (First Embodiment)

### (Information Provision System)

An information provision system according to a first embodiment of the present invention will be described with reference to drawings.

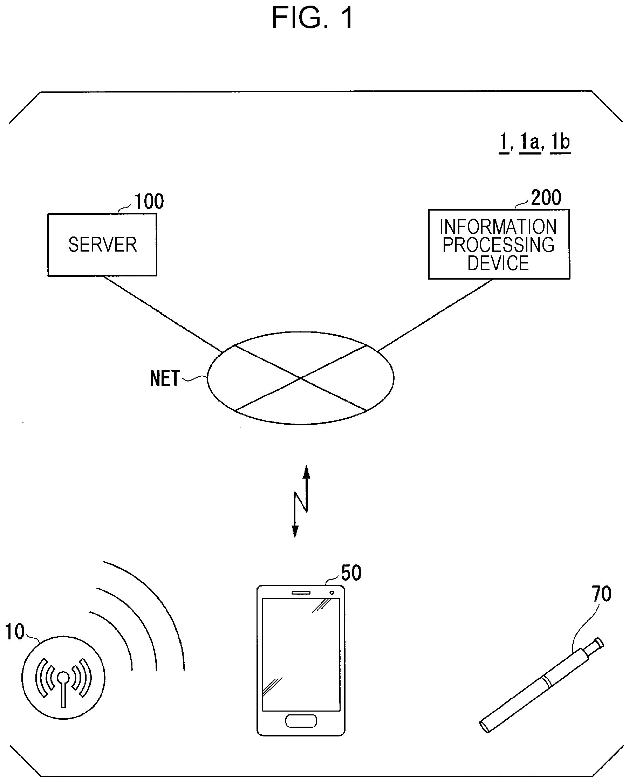

Fig. 1 is a diagram illustrating an example of the information provision system according to the first embodiment. An information provision system 1 according to this embodiment includes a beacon transmission device 10, a user terminal 50, an aerosol generation device 70, a server 100, and an information processing device 200. The user terminal 50, the server 100, and the information processing device 200 are connected to one another via a communication network NET. The communication network NET includes, for example, a wireless base station, a Wi-Fi access point, a communication link, a provider, the Internet, and the like. The communication network NET may partially include a local network. The user terminal may be represented by a terminal device.

The communication network NET may be, for example, a wired network or a wireless network. The communication network NET may be, for example, Wi-Fi (registered trademark), an ad hoc network, an intranet, an extranet, a virtual private network (VPN), a local area network (LAN), a wireless LAN (WLAN), a wide area network (WAN), a wireless WAN (WWAN), a metropolitan area network (MAN), part of the Internet, a public switched telephone network (PSTN), integrated service digital networks (ISDNs), long term evolution (LTE), code division multiple access (CDMA), Bluetooth (registered trademark), Bluetooth low energy (BLE), a low power wide area network (LPWAN), or the like. Furthermore, the communication network NET may be satellite communication. The communication network NET may be, for example, wired communication such as a universal serial bus (USB), a Mini USB, a Micro USB, or Lightning. The communication network NET may be a combination of the communication methods mentioned above. However, the communication network NET is not limited to any of the communication methods mentioned above.

Reception, by the user terminal 50 held by a user of the aerosol generation device 70, of a beacon signal transmitted from the beacon transmission device 10 triggers the information provision system 1 to provide information pertaining to the aerosol generation device 70 to the user terminal 50.

The beacon transmission device 10 transmits a beacon signal including a beacon ID at a specific cycle. The beacon ID is an identifier that can uniquely identify the beacon signal. The predetermined cycle may be any length of cycle such as a cycle of one second or a cycle of five seconds. For example, the beacon transmission device 10 may be installed in a place where smoking is permitted, such as a smoking area, a shop, or the like. In this embodiment, for example, a case where the beacon transmission device 10 is installed in a smoking area will be described below.

The user terminal 50 stores a user ID of the user of the aerosol generation device 70. The user ID is an identifier that can uniquely identify the user. The user terminal 50 stores a beacon ID in association with the address of the server 100. The user terminal 50 receives a beacon signal transmitted from the beacon transmission device 10, and creates identification information which includes the beacon ID included in the received beacon signal and the user ID and which is addressed to the address of the server 100, which is stored in association with the beacon ID. The user terminal 50 transmits the created identification information to the server 100.

For example, the aerosol generation device 70 is a device that atomizes liquid (an aerosol source) by using electric power to generate an aerosol. Aerosols are obtained by atomizing the aerosol source and are particles fine enough to float in the air. An aerosol generated by the aerosol generation device 70 may be accompanied with flavor and taste. Examples of the aerosol generation device 70 include a heated tobacco product (T-vapor or Infused), an electronic cigarette product (E-vapor), and the like. The aerosol generation device 70 also includes a type of directly heating tobacco (direct heat), a type of indirectly heating tobacco (indirect heat), and a type of heating liquid (liquid). The aerosol generation device 70 may atomize liquid by generating surface acoustic waves (SAWs) by using a piezoelectric element substrate including a comb-shaped electrode pair. As described above, the aerosol generation device 70 includes various devices such as a combustion-type device, a non-combustion-type device, a heat-type device, and a non-heat-type device.

In the case where the aerosol generation device 70 is of a type that generates SAWs by using a piezoelectric element substrate including a comb-shaped electrode pair to atomize liquid, the aerosol generation device 70 includes, for example, as atomizing unit, a piezoelectric element substrate including a comb-shaped electrode pair and a liquid supply part configured to supply a liquid aerosol source to the piezoelectric element substrate. The piezoelectric element substrate may be configured to atomize an aerosol source by using surface acoustic waves generated by applying voltage at a high frequency to the comb-shaped electrode pair. Furthermore, the piezoelectric element substrate includes comb-shaped electrode pairs whose number is set on the basis of desired aerosols to be obtained by atomization using surface acoustic waves. The number of the comb-shaped electrode pairs is set on the basis of desired aerosols. Thus, by improving the efficiency of atomization of an aerosol source in the atomizing unit that can supply a limited amount of electric power to a comb-shaped electrode pair, an appropriate atomizing unit can be provided.

The device described above is merely an example. The aerosol generation device 70 may be any device that generates an aerosol.

The server 100 is included in a message exchange system capable of transmitting a message to the user terminal 50. The server 100 stores a beacon ID, the address of the information processing device 200, and reception location information in association with one another. The reception location information is information pertaining to the location where the beacon signal is received. Information regarding a smoking area where the beacon transmission device 10 that has transmitted the beacon signal is installed is an example of the information pertaining to the location where the beacon signal is received.

The server 100 receives the identification information transmitted from the user terminal 50, and thus acquires the beacon ID and the user ID that are included in the received identification information. The server 100 also acquires the address of the information processing device 200 and the reception location information that are associated with the acquired beacon ID. Furthermore, the server 100 creates a location information notification which includes the acquired user ID and reception location information and which is addressed to the address of the information processing device 200, and transmits the created location information notification to the information processing device 200.

The information processing device 200 stores the user ID, the reception location information, and provision information in association with one another. The provision information represents information such as advertising relating to the aerosol generation device 70 that is held by the user corresponding to the user ID, notes, and the like. Advertising relating to the body and flavor of the aerosol generation device 70, information of a new product of the aerosol generation device 70, and the like are examples of the advertising relating to the aerosol generation device 70. The information relating to the aerosol generation device 70 may vary according to the reception location information.

The information processing device 200 receives the location information notification transmitted from the server 100, and thus acquires the user ID and the reception location information that are included in the received location information notification. The information processing device 200 acquires the provision information that is stored in association with the combination of the acquired user ID and reception location information. The information processing device 200 creates a provision information notification which includes the acquired provision information and user ID and which is addressed to the address of the server 100, and transmits the created provision information notification to the server 100.

The server 100 receives the provision information notification transmitted from the information processing device 200, and thus acquires the provision information and the user ID that are included in the received provision information notification. The server 100 creates information to be notified to the user (hereinafter, called "user notification information") which includes the acquired provision information and which is addressed to the address of the user terminal 50 corresponding to the user ID, and transmits the created user notification information to the user terminal 50.

The user terminal 50 receives the user notification information transmitted from the server 100, processes the provision information included in the received user notification information, and thus provides information to the user. Hereinafter, the aerosol generation device 70, the user terminal 50, the server 100, and the information processing device 200 that are included in the information provision system 1 will be described.

### (Aerosol Generation Device)

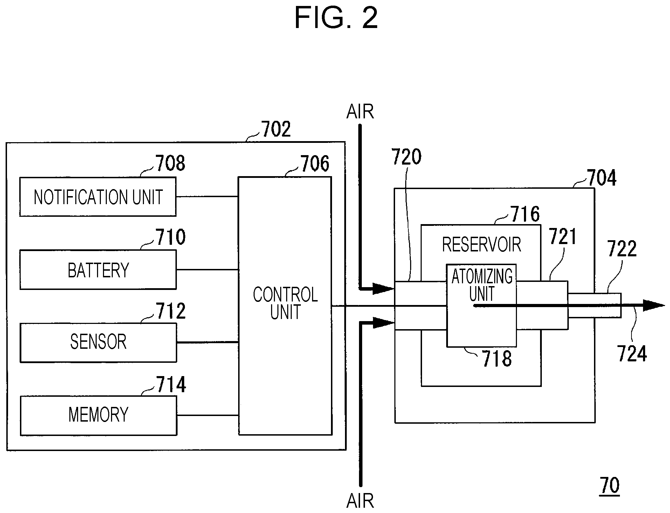

Fig. 2 is a block diagram illustrating a schematic configuration of an aerosol generation device according to the first embodiment.

As illustrated in Fig. 2, the aerosol generation device 70 includes a first member 702 and a second member 704. As illustrated in the drawing, for example, the first member 702 may include a control unit 706, a notification unit 708, a battery 710, a sensor 712, and a memory 714. For example, the second member 704 may include a reservoir 716, an atomizing unit 718, an air intake flow passage 720, an aerosol flow passage 721, and a mouthpiece unit 722. Part of the components included in the first member 702 may be included in the second member 704. Part of the components included in the second member 704 may be included in the first member 702. The second member 704 may be configured to be detachable from the first member 702. Alternatively, all the components included in the first member 702 and the second member 704 may be included in the same housing in place of the first member 702 and the second member 704.

The reservoir 716 holds an aerosol source. For example, the reservoir 716 is formed of fibrous or porous material and holds an aerosol source as liquid in gaps between fibers or pores of the porous material. As the fibrous or porous material mentioned above, for example, cotton, glass fibers, a tobacco raw material, or the like may be used. The reservoir 716 may be configured to be a tank that stores liquid. The aerosol source is, for example, liquid such as water or polyhydric alcohol such as glycerol or propylene glycol. In the case where the aerosol generation device 70 is a medical inhaler such as a nebulizer, the aerosol source may contain a medicine to be inhaled by a patient. As another example, the aerosol source may contain a tobacco raw material emitting a flavor smoke taste component when heated or an extract derived from a tobacco raw material. The reservoir 716 may be configured such that a consumed aerosol source can be replenished. Alternatively, the reservoir 716 may be configured such that the reservoir 716 itself can be replaced after the aerosol source is consumed. The aerosol source is not necessarily liquid and may be solid. In the case where the aerosol source is solid, the reservoir 716 may be, for example, a hollow container that is not made of a fibrous or porous material.

The atomizing unit 718 is configured to atomize an aerosol source to generate an aerosol. When an inhale operation is detected by the sensor 712, the atomizing unit 718 generates an aerosol. For example, a wick (not illustrated in the drawing) may be provided to connect the reservoir 716 to the atomizing unit 718. In this case, part of the wick extends inside the reservoir 716 to be in contact with the aerosol source. Another part of the wick extends to the atomizing unit 718. Due to capillarity of the wick, the aerosol source is transported to the atomizing unit 718 from the reservoir 716. For example, the atomizing unit 718 includes a heater that is electrically connected to the battery 710. The heater is arranged to be in contact with or adjacent to the wick. When the inhale operation is detected, the control unit 706 controls the heater of the atomizing unit 718 to heat the aerosol source transported through the wick, so that the aerosol source is atomized. Another example of the atomizing unit 718 may be an ultrasonic atomizer that atomizes an aerosol source by using ultrasound vibrations. The air intake flow passage 720 is connected to the atomizing unit 718, and the air intake flow passage 720 extends outside the aerosol generation device 70. The aerosol generated by the atomizing unit 718 is mixed with air taken in through the air intake flow passage 720. The mixed fluid of the aerosol and the air is sent out into the aerosol flow passage 721, as indicated by arrow 724. The aerosol flow passage 721 has a tubular structure for transporting the mixed fluid of the aerosol and the air generated by the atomizing unit 718 to the mouthpiece unit 722.

The mouthpiece unit 722 is located at the end edge of the aerosol flow passage 721 and is configured such that the aerosol flow passage 721 is opened to the outside of the aerosol generation device 70. When a user inhales with the mouthpiece unit 722 held in his/her mouth, the user takes air containing the aerosol into his/her oral cavity.

The notification unit 708 may include a light-emitting element such as an LED, a display, a speaker, a vibrator, and the like. The notification unit 708 is configured to provide a notification to the user by light emission, display, producing sound, vibration, or the like as needed.

The battery 710 supplies electric power to each of the components of the aerosol generation device 70 including the notification unit 708, the sensor 712, the memory 714, and the atomizing unit 718. The battery 710 can be charged by being connected to an external power source via a predetermined port (not illustrated in the drawing) of the aerosol generation device 70. Only the battery 710 may be detachable from the first member 702 or the aerosol generation device 70 or the battery 710 may be replaced with a new battery 710. Furthermore, the battery 710 may be replaced with a new battery 710 by replacing the entire first member 702 with a new first member 702.

The sensor 712 may include a pressure sensor that detects variations in the pressure inside the air intake flow passage 720 and/or the aerosol flow passage 721 or a flow rate sensor that detects the flow rate inside the air intake flow passage 720 and/or the aerosol flow passage 721. The sensor 712 may also include a weight sensor that detects the weight of a component such as the reservoir 716. Furthermore, the sensor 712 may be configured to count the number of puffs by the user by using the aerosol generation device 70. The sensor 712 may also be configured to accumulate times during which the atomizing unit 718 is electrically connected. The sensor 712 may further be configured to detect the height of the liquid level in the reservoir 716. The sensor 712 may also be configured to detect the state of charge (SOC), integrated current value, voltage, and the like of the battery 710. The integrated current value may be obtained by a current integration method, SOC-OCV (open circuit voltage) method, or the like. The sensor 712 may be an operation button that may be operated by the user.

The control unit 706 may be an electronic circuit module configured to be a microprocessor or a microcomputer. The control unit 706 may be configured to control an operation of the aerosol generation device 70 in accordance with a computer-executable instruction stored in the memory 714. The memory 714 is a storage medium such as a ROM, a RAM, or a flash memory. In addition to the above-mentioned computer-executable instruction, setting data and the like necessary for control of the aerosol generation device 70 may be stored in the memory 714. For example, various data including a control method for the notification unit 708 (modes such as light emission, producing sound, and vibration), values detected by the sensor 712, and heating history for the atomizing unit 718 may be stored in the memory 714. The control unit 706 reads data from the memory 714 as needed and uses the read data for control of the aerosol generation device 70. The control unit 706 also stores data into the memory 714 as needed.

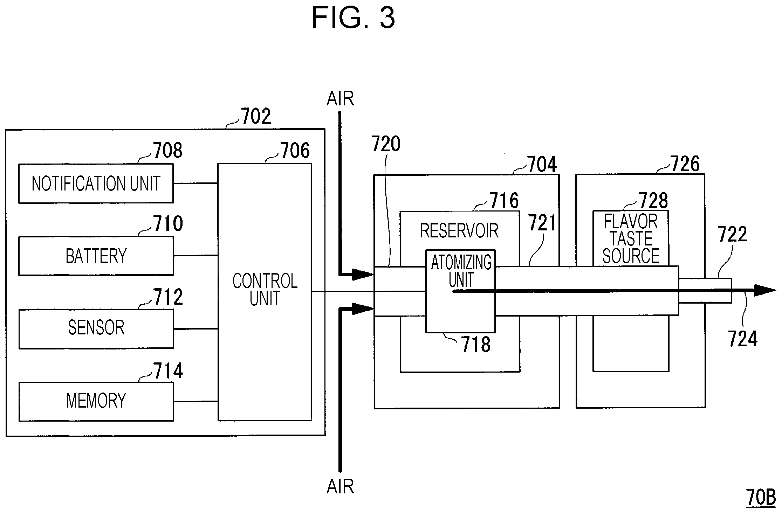

Fig. 3 is a block diagram illustrating another schematic configuration of an aerosol generation device according to the first embodiment.

As illustrated in the drawing, an aerosol generation device 70B includes a third member 726, in addition to the configuration of the aerosol generation device 70 in Fig. 2. The third member 726 may include a flavor taste source 728. For example, in the case where the aerosol generation device 70B is an electronic cigarette, the flavor taste source 728 may contain a flavor smoke taste component contained in tobacco. As illustrated in the drawing, the aerosol flow passage 721 extends over the second member 704 and the third member 726. The mouthpiece unit 722 is provided at the third member 726.

The flavor taste source 728 is a component for adding flavor taste to an aerosol. The flavor taste source 728 is arranged in the middle of the aerosol flow passage 721. The mixed fluid of an aerosol and air generated by the atomizing unit 718 (hereafter, note that the mixed fluid may be simply called an aerosol) flows through the aerosol flow passage 721 and reaches the mouthpiece unit 722. As described above, the flavor taste source 728 is arranged downstream the atomizing unit 718 in the flow of the aerosol. In other words, the flavor taste source 728 is located closer to the mouthpiece unit 722 in the aerosol flow passage 721 than the atomizing unit 718 is. Thus, the aerosol generated by the atomizing unit 718 passes through the flavor taste source 728 and reaches the mouthpiece unit 722. The flavor smoke taste component contained in the flavor taste source 728 is added to the aerosol that is passing through the flavor taste source 728. For example, in the case where the aerosol generation device 70B is an electronic cigarette, the flavor taste source 728 may be derived from tobacco, such as shredded tobacco or a processed item obtained by forming a tobacco raw material into a granular, sheet, or powder state. Furthermore, the flavor taste source 728 may be derived from a non-tobacco material such as plants (for example, mint, herb, etc.) other than tobacco. For example, the flavor taste source 728 contains a nicotine component. The flavor taste source 728 may contain a flavor component such as menthol. In addition to the flavor taste source 728, the reservoir 716 may also contain a substance containing a flavor smoke taste component. For example, the aerosol generation device 70B may be configured such that the flavor taste source 728 holds a flavor taste substance derived from tobacco and the reservoir 716 contains a flavor taste substance not derived from tobacco.

The user is able to take the air containing the aerosol, to which flavor taste has been added, into his/her oral cavity by inhaling with the mouthpiece unit 722 held in his/her mouth.

The control unit 706 is configured to control the aerosol generation devices 70 and 70B according to an embodiment of the present disclosure (hereinafter, may collectively be referred to as "aerosol generation devices 70) in various methods.

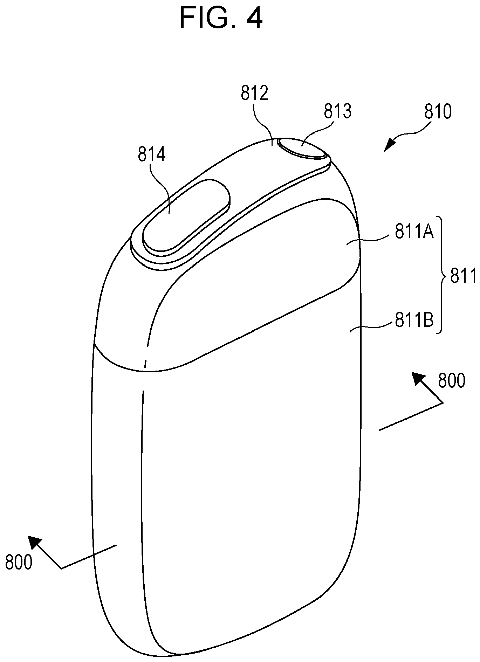

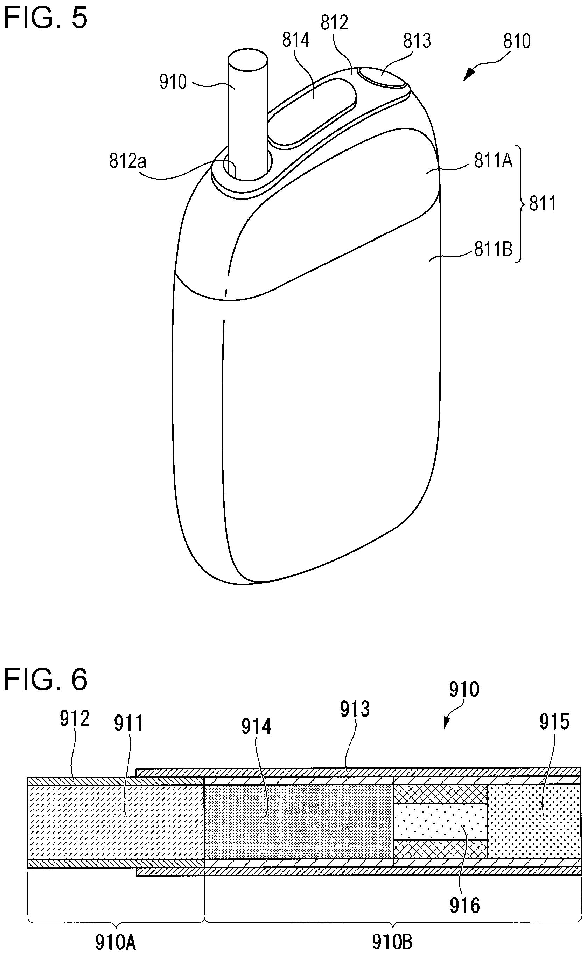

Fig. 4 is an entire perspective view of an aerosol generation device according to the first embodiment. Fig. 5 is an entire perspective view of another aerosol generation device according to the first embodiment that is holding an aerosol-source material. In this embodiment, for example, the aerosol generation device 70 is configured to generate an aerosol containing flavor taste by heating an aerosol-source material such as a smoking article containing a flavor taste source material such as a filler including an aerosol source and a flavor taste source. A smoking article 910 may be used as an aerosol-source material.

As understood by those skilled in the art, the smoking article 910 is merely an example of an aerosol-source material. The aerosol source contained in the aerosol-source material may be solid or liquid. The aerosol source may be, for example, liquid such as water or polyhydric alcohol such as glycerol or propylene glycol. The aerosol source may contain a tobacco raw material emitting a flavor smoke taste component when heated or an extract derived from the tobacco raw material. In the case where the aerosol generation device 70 is a medical inhaler such as a nebulizer, the aerosol source may contain a medicine to be inhaled by a patient. Depending on the purpose of use, the aerosol-source material does not necessarily contain a flavor taste source.

As illustrated in Fig. 4 and 5, the aerosol generation device 70 includes a top housing 811A, a bottom housing 811B, a cover 812, a switch 813, and a lid part 814. The top housing 811A and the bottom housing 811B are connected to each other to form an outermost housing 811 of the aerosol generation device 70. The housing 811 may be of a size that is small enough to be held in a hand of the user. In this case, in using the aerosol generation device 70, the user is able to inhale an aerosol while holding the aerosol generation device 70 in his/her hand.

The top housing 811A has an opening (not illustrated in the drawing), and the cover 812 is bonded to the top housing 811A so as to cover the opening. As illustrated in Fig. 5, the cover 812 has an opening 812a into which the smoking article 910 can be inserted. The lid part 814 is configured to open and close the opening 812a of the cover 812. Specifically, the lid part 814 is attached to the cover 812 and is configured to be able to move between a first position for closing the opening 812a and a second position for opening the opening 812a along the surface of the cover 812.

The switch 813 is used to switch between ON and OFF of operation of the aerosol generation device 70. For example, as illustrated in Fig. 5, when the user operates the switch 813 with the smoking article 910 inserted in the opening 812a, electric power is supplied from a battery (not illustrated in the drawing) to a heating unit (not illustrated in the drawing), so that the smoking article 910 can be heated without being burned. When the smoking article 910 is heated, an aerosol is generated from the aerosol source contained in the smoking article 910, and flavor taste of the flavor taste source is taken into the aerosol. The user sucks a part of the smoking article 910 (part illustrated in Fig. 5) that protrudes from the aerosol generation device 70, and is thus able to inhale the aerosol containing the flavor taste. A direction in which the aerosol-source material such as the smoking article 910 is inserted in the opening 812a will be hereinafter referred to as a longitudinal direction of the aerosol generation device 70.

The configuration of the aerosol generation device 70 illustrated in Figs. 4 and 5 is merely an example of a configuration of an aerosol generation device according to the present disclosure. An aerosol generation device according to the present disclosure may be configured in various modes in which the aerosol generation device is able to generate an aerosol by heating an aerosol-source material containing an aerosol source and a user is able to inhale the generated aerosol source.

Next, a configuration of the smoking article 910 will be described as an example of an aerosol-source material used for the aerosol generation device 70 according to this embodiment. Fig. 6 is a sectional view of the smoking article 910. In an embodiment illustrated in Fig. 6, the smoking article 910 includes a base material unit 910A including a filler 911 (corresponding to an example of a flavor taste source material) and first wrapping paper 912 that wraps the filler 911, and a mouthpiece unit 910B that forms an end part opposite the base material unit 910A. The base material unit 910A and the mouthpiece unit 910B are connected by second wrapping paper 913 that is different from the first wrapping paper 912. However, the second wrapping paper 913 may be omitted, and the base material unit 910A and the mouthpiece unit 910B may be connected by the first wrapping paper 912.

The mouthpiece unit 910B in Fig. 6 includes a paper tube part 914, a filter part 915, and a hollow segment part 916 arranged between the paper tube part 914 and the filter part 915. The hollow segment part 916 includes, for example, a filling layer including one or more hollow channels and a plug wrapper wrapping the filling layer. Due to the high fiber packing density in the filling layer, when the user inhales, air and an aerosol flow only in the hollow channel(s), and only little air and aerosol flow in the filling layer. In the smoking article 910, reducing the length of the filter part 915 and replacing the reduced part of the filter part 915 with the hollow segment part 916 in order to suppress a decrease in the amount of aerosol delivered caused by filtration of an aerosol component at the filter part 915 is effective for increasing the amount of aerosol delivered.

In the embodiment in Fig. 6, the mouthpiece unit 910B is composed of three segments. However, in another embodiment, the mouthpiece unit 910B may be composed of one or two segments or may be composed of four or more segments. For example, the mouthpiece unit 910B may be formed such that the hollow segment part 916 is omitted and the paper tube part 914 and the filter part 915 are arranged adjacent to each other.

In the embodiment illustrated in Fig. 6, the length of the smoking article 910 in the longitudinal direction ranges preferably from 40 mm to 90 mm, more preferably from 50 mm to 75 mm, and still more preferably from 50 mm to 60 mm The circumference of the smoking article 910 ranges preferably from 15 mm to 25 mm, more preferably from 17 mm to 24 mm or less, and still more preferably from 20 mm to 22 mm Furthermore, the length of the base material unit 910A of the smoking article 910 may be 20 mm, the length of the first wrapping paper 912 may be 20 mm, the length of the hollow segment part 916 may be 8 mm, and the length of the filter part 915 may be 7 mm The length of each of the segments may be changed appropriately according to the manufacturing suitability, required quality, and the like.

In this embodiment, the filler 911 of the smoking article 910 may contain an aerosol source that generates an aerosol when heated at a predetermined temperature. The type of aerosol source is not particularly limited. A material extracted from various natural products and/or constituent components of the natural products may be selected according to the purpose of use. As an aerosol source, for example, glycerol, propylene glycol, triacetin, 1,3-butanediol, or a mixture of these components may be used. The aerosol source content in the filler 911 is not particularly limited. From the viewpoint of sufficient generation of aerosol and provision of good flavor smoke taste, the aerosol source content in the filler 911 is normally 5 percent or more by weight, preferably 10 percent or more by weight, and normally 50 percent or less by weight, preferably 20 percent or less by weight.

The filler 911 of the smoking article 910 in this embodiment may contain tobacco shreds as a flavor taste source. Materials of tobacco shreds are not particularly limited. Known materials such as lamina or midrib may be used. In the case where the circumference is 22 mm and the length is 20 mm, the filler 911 content in the smoking article 910 ranges, for example, from 200 mg to 400 mg, preferably from 250 mg to 320 mg. The moisture content of the filler 911 ranges, for example, from 8 percent by weight to 18 percent by weight, preferably from 10 percent by weight to 16 percent by weight. With the above moisture content, occurrence of stains on wrapping can be reduced, and excellent machinability during the production of the base material unit 910A can be achieved. The size of tobacco shreds used as the filler 911 and a preparation method for tobacco shreds are not particularly limited. For example, dried tobacco leaves shredded to a width ranging from 0.8 mm to 1.2 mm may be used. Furthermore, dried tobacco leaves that have been uniformly grounded to an average particle diameter ranging from about 20 µm to 200 µm may be processed into a sheet, and the sheet may be shredded to a width ranging from 0.8 mm to 1.2 mm and used. Furthermore, the sheet obtained by the processing mentioned above may be gathered without being shredded and the gathered sheet may be used as the filler 911. The filler 911 may have a single flavor or two or more flavors. The type of flavor is not particularly limited. From the viewpoint of provision of an excellent smoke taste, a flavor is preferably menthol.

In this embodiment, the first wrapping paper 912 and the second wrapping paper 913 of the smoking article 910 may be made from base paper with a basis weight ranging, for example, from 20 gsm to 65 gsm, preferably from 25 gsm to 45 gsm. The thickness of the first wrapping paper 912 and the second wrapping paper 913 is not particularly limited. However, from the viewpoint of stiffness, air permeability, and easiness of adjustment at the time of paper making, the thickness of the first wrapping paper 912 and the second wrapping paper 913 ranges from 10 µm to 100 µm, preferably from 20 µm to 75 µm, and still more preferably from 30 µm to 50 µm.

In this embodiment, the first wrapping paper 912 and the second wrapping paper 913 of the smoking article 910 may contain filler. The filler content may be 10 percent or more by weight and 60 percent or less by weight, preferably range from 15 percent by weight to 45 percent by weight, of the entire weight of the first wrapping paper 912 and the second wrapping paper 913. In this embodiment, the filler ranges preferably from 15 percent by weight to 45 percent by weight of a preferable range of basis weight (25 gsm to 45 gsm). As the filler, for example, calcium carbonate, titanium dioxide, kaolin, or the like may be used. Paper containing such filler can exhibit whitish bright color, which is desirable from the viewpoint of appearance when used as wrapping paper for the smoking article 910, and permanently maintain brightness. For example, wrapping paper containing much filler as described above can achieve an ISO brightness of 83 percent or more. Furthermore, from the practical point of view of using the first wrapping paper 912 and the second wrapping paper 913 as wrapping paper for the smoking article 910, the first wrapping paper 912 and the second wrapping paper 913 preferably have a tensile strength of 8N/15 mm or more. The tensile strength can be increased by reducing the filler content. Specifically, the tensile strength can be increased by setting the filler content to lower than the upper limit of the filler content in the range of basis weight in the example mentioned above.

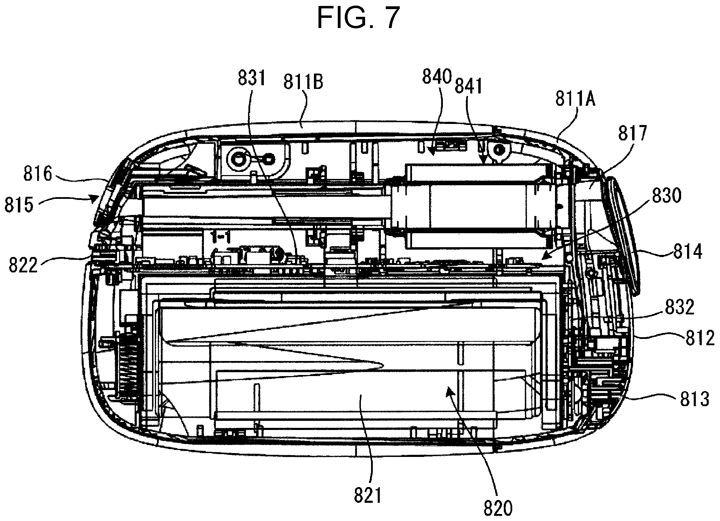

Next, the internal structure of the aerosol generation device 70 illustrated in Figs. 4 and 5 will be described. Fig. 7 is a sectional view taken along arrow 800-800 illustrated in Fig. 4. As illustrated in Fig. 7, the aerosol generation device 70 includes a power supply unit 820, a circuit unit 830, and a heating unit 840 in an inner space of the housing 811. The circuit unit 830 may include a first circuit substrate 831 and a second circuit substrate 832 that is electrically connected to the first circuit substrate 831. The first circuit substrate 831 may be arranged, for example, to extend in the longitudinal direction, as illustrated in the drawing. Accordingly, the power supply unit 820 and the heating unit 840 are partitioned by the first circuit substrate 831. As a result, transmission of heat generated at the heating unit 840 to the power supply unit 820 can be reduced.

The second circuit substrate 832 may be arranged between the top housing 811A and the power supply unit 820 or may extend in the direction orthogonal to the direction in which the first circuit substrate 831 extends. The switch 813 may be arranged adjacent to the second circuit substrate 832. When the user presses the switch 813, part of the switch 813 may be in contact with the second circuit substrate 832.

The first circuit substrate 831 and the second circuit substrate 832 each include, for example, a microprocessor or the like and can control electric power supplied from the power supply unit 820 to the heating unit 840. Accordingly, the first circuit substrate 831 and the second circuit substrate 832 can control heating of the smoking article 910 by the heating unit 840.

The power supply unit 820 includes a power supply 821 that is electrically connected to the first circuit substrate 831 and the second circuit substrate 832. The power supply 821 may be, for example, a rechargeable or non-rechargeable battery. The power supply 821 is electrically connected to the heating unit 840 via at least one of the first circuit substrate 831 and the second circuit substrate 832. Accordingly, the power supply 821 can supply electric power to the heating unit 840 so that the smoking article 910 can be properly heated. Furthermore, as illustrated in the drawing, the power supply 821 may be arranged adjacent in the direction orthogonal to the longitudinal direction of the heating unit 840. Thus, the length of the aerosol generation device 70 in the longitudinal direction can be prevented from increasing even in the case where the size of the power supply 821 increases.

Furthermore, the aerosol generation device 70 may include a terminal 822 that can be connected to an external power source. The terminal 822 can be connected to a cable such as a Micro USB. In the case where the power supply 821 is a rechargeable battery, when an external power source is connected to the terminal 822, electric current flows to the power supply 821 from the external power source, so that the power supply 821 can be charged. Furthermore, a data transmission cable such as a Micro USB may be connected to the terminal 822 so that data pertaining to operation of the aerosol generation device 70 can be transmitted to an external device.

The heating unit 840 includes, as illustrated in the drawing, a heating assembly 841 that extends in the longitudinal direction. The heating assembly 841 includes a plurality of cylindrical members to form a cylindrical body as a whole. The heating assembly 841 is configured to be able to house part of the smoking article 910 and includes a function for defining a passage for air to be supplied to the smoking article 910 and a function for heating the smoking article 910 from its outer periphery.

The bottom housing 811B has a vent 815 through which air flows into the heating assembly 841. Specifically, the vent 815 fluidically communicates with one end part (a left end part in Fig. 6) of the heating assembly 841. Furthermore, the aerosol generation device 70 includes a cap 816 that is detachable from the vent 815. The cap 816 is configured such that even in a state in which the cap 816 is placed over the vent 815, air flows into the heating assembly 841 through the vent 815, and the vent 815 may have, for example, a through-hole or a notch, which is not illustrated in the drawing. By attaching the cap 816 to the vent 815, a substance generated from the smoking article 910 inserted in the heating assembly 841 can be prevented from falling out of the housing 811 through the vent 815. Furthermore, by removing the cap 816, an area inside the heating assembly 841 or inside the cap 816 can be cleaned.

The other end part (a right end part in Fig. 6) of the heating assembly 841 fluidically communicates with the opening 812a illustrated in Fig. 5. An outer fin 817 of a substantially cylindrical shape is provided between the lid part 814 having the opening 812a and the other end part of the heating assembly 841. As illustrated in Fig. 5, the smoking article 910 that is inserted into the aerosol generation device 70 through the opening 812a of the lid part 814, as illustrated in Fig. 5, passes through the outer fin 817, and part of the smoking article 910 is arranged inside the heating assembly 841. Thus, it is preferable that the outer fin 817 be formed such that the size of the opening near the lid part 814 is larger than the size of the opening near the other end part of the heating assembly 841. Thus, the smoking article 910 can be easily inserted into the outer fin 817 through the opening 812a.

When the user inhales through the part of the smoking article 910 that protrudes from the aerosol generation device 70, that is, the filter part 915 illustrated in Fig. 6, with the smoking article 910 inserted in the aerosol generation device 70 through the opening 812a, as illustrated in Fig. 5, air flows into the heating assembly 841 through the vent 815. The flowing air passes through inside the heating assembly 841 and reaches inside the mouth of the user, along with an aerosol generated by the smoking article 910. Thus, a side of the heating assembly 841 that is closer to the vent 815 is defined as an upstream side, and a side of the heating assembly 841 that is closer to the opening 812a (closer to the outer fin 817) is defined as a downstream side.

### (User Terminal)

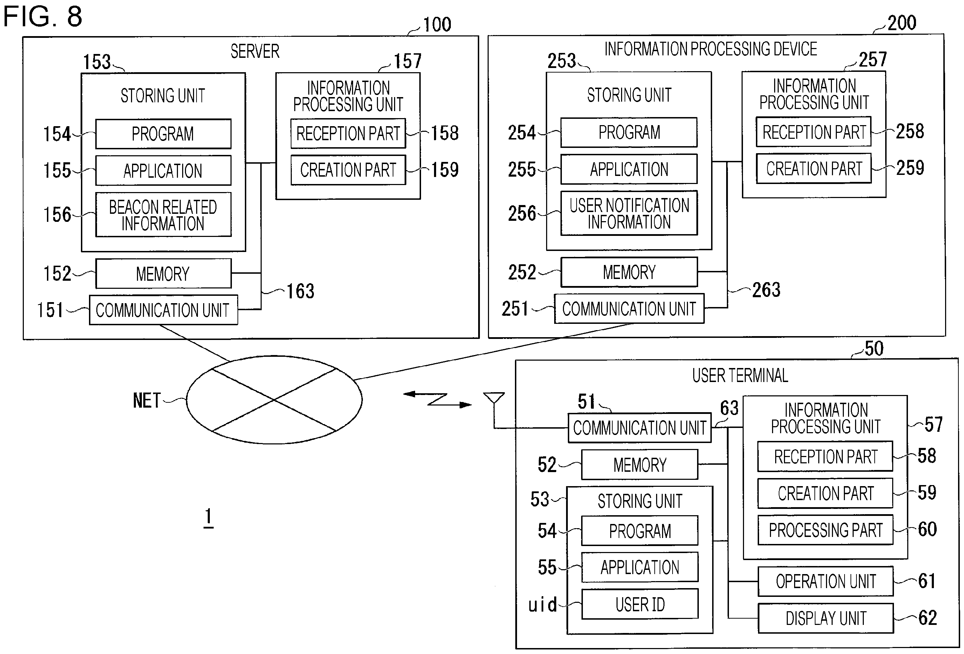

Fig. 8 is a block diagram illustrating an example of an information provision system according to the first embodiment. The user terminal 50 is implemented by a smartphone, a portable terminal, a personal computer, a tablet terminal device, a wristwatch-type terminal device, a glasses-type terminal device, or other types of information processing equipment. Furthermore, the user terminal 50 may be a smart speaker (AI speaker: "AI" is an abbreviation of "artificial intelligence") or a head mount display (HMD: Head Mounted Display). However, the user terminal 50 is not limited to the examples mentioned above. The user terminal 50 includes a communication unit 51, a memory 52, a storing unit 53, an information processing unit 57, an operation unit 61, a display unit 62, and a bus line 63 such as an address bus or a data bus for electrically connecting the components of the user terminal 50, as illustrated in Fig. 8. The user terminal may be represented as a terminal device.

The communication unit 51 is implemented by a communication module. Specifically, the communication unit 51 is configured to be a wireless device that performs wireless communication using a wireless communication technique such as a wireless LAN (registered trademark) or LTE. Furthermore, the communication unit 51 may be configured to be a device that performs wired communication. The communication unit 51 communicates with an external communication device such as the server 100 via the communication network NET. Specifically, the communication unit 51 receives a beacon signal transmitted from the beacon transmission device 10, and outputs the received beacon signal to the information processing unit 57. The communication unit 51 acquires identification information output from the information processing unit 57, and transmits the acquired identification information to the server 100. Furthermore, the communication unit 51 receives user notification information transmitted from the server 100, and outputs the received user notification information to the information processing unit 57.

The memory 52 includes a RAM such as a volatile memory including a semiconductor element and is used as a work memory for the information processing unit 57. The memory 52 includes, for example, a flash memory or the like.

The storing unit 53 is implemented by, for example, a random access memory (RAM), a read only memory (ROM), a hard disk drive (HDD), a flash memory, a hybrid storage device including a combination of some of the RAM, the ROM, the HDD, and the flash memory, or the like. A program 54 and an application 55 that are to be executed by the information processing unit 57 are stored in the storing unit 53. Furthermore, a user ID uid is stored in the storing unit 53.

The program 54 is, for example, an operating system and is positioned between a user or an application program and hardware. The program 54 provides a standard interface to the user or the application program and, at the same time, manages resources such as the hardware efficiently.

The application 55 causes the user terminal 50 to receive a beacon signal transmitted from the beacon transmission device 10. The application 55 causes the user terminal 50 to create identification information which includes a beacon ID included in the received beacon signal and a user ID and which is addressed to the address of the server 100 that is associated with the beacon ID. The application 55 causes the user terminal 50 to transmit the created identification information to the server 100. The application 55 causes the user terminal 50 to receive user notification information transmitted from the server 100. The application 55 causes the user terminal 50 to process provision information included in the received user notification information and thus provide information to the user.

The user ID uid is an identifier that can uniquely identify the user of the user terminal 50.

The operation unit 61 includes, for example, a touch panel or the like. The operation unit 61 detects a touch operation on a screen displayed on the display unit 62, and outputs a result of detection of the touch operation to the information processing unit 57.

The display unit 62 displays information to be provided to the user.

The entire or part of the information processing unit 57 is a functional unit (hereinafter, referred to as a software functional unit) that is implemented when, for example, a processor such as a central processing unit (CPU) executes the program 54 and the application 55 stored in the storing unit 53. The entire or part of the information processing unit 57 may be implemented by hardware such as a large scale integration (LSI), an application specific integrated circuit (ASIC), or a field-programmable gate array (FPGA) or may be implemented by a combination of the software functional unit and the hardware.

The information processing unit 57 includes, for example, a reception part 58, a creation part 59, and a processing part 60.

The reception part 58 acquires the beacon signal output from the communication unit 51, and receives the acquired beacon signal. The reception part 58 outputs the received beacon signal to the creation part 59. The reception part 58 acquires the user notification information output from the communication unit 51, and receives the acquired user notification information. The reception part 58 outputs the received user notification information to the processing part 60.

The creation part 59 acquires the beacon signal output from the reception part 58, and acquires a beacon ID included in the acquired beacon signal. When acquiring the beacon ID included in the beacon signal, the creation part 59 acquires the user ID uid stored in the storing unit 53, and creates identification information which includes the acquired user ID uid and beacon ID and which is addressed to the address of the server 100 that is associated with the beacon ID. The creation part 59 outputs the created identification information to the communication unit 51.

The processing part 60 acquires the user notification information output from the reception part 58, processes the acquired user notification information, and displays a result of the processing on the display unit 62. As a result, information such as advertising relating to the aerosol generation device 70 and notes are displayed on the display unit 62.

### (Server)

The server 100 is implemented by a device such as a personal computer or an industrial computer. However, the server 100 is not limited to the examples mentioned above. The server 100 includes a communication unit 151, a memory 152, a storing unit 153, an information processing unit 157, and a bus line 163 such as an address bus or a data bus for electrically connecting the components of the server 100 as illustrated in Fig. 8.

The communication unit 151 is implemented by a communication module. Specifically, the communication unit 51 is configured to be a device that performs wired communication. Furthermore, the communication unit 151 may be configured to be a wireless device that performs wireless communication using a wireless communication technique such as a wireless LAN (registered trademark). The communication unit 151 communicates with external communication devices such as the user terminal 50 and the information processing device 200 via the communication network NET.

Specifically, the communication unit 151 receives identification information transmitted from the user terminal 50, and outputs the received identification information to the information processing unit 157. The communication unit 151 acquires reception location information output from the information processing unit 157, and transmits the acquired reception location information to the information processing device 200. The communication unit 151 receives a provision information notification transmitted from the information processing device 200, and outputs the received provision information notification to the information processing unit 157. The communication unit 151 acquires user notification information output from the information processing unit 157, and transmits the acquired user notification information to the user terminal 50.

The memory 152 includes a RAM such as a volatile memory including a semiconductor element and is used as a work memory for the information processing unit 157. The memory 152 includes, for example, a flash memory or the like.

The storing unit 153 is implemented by, for example, a RAM, a ROM, an HDD, a flash memory, a hybrid storage device including a combination of some of the RAM, the ROM, the HDD, and the flash memory, or the like. A program 154 and an application 155 that are to be executed by the information processing unit 157 are stored in the storing unit 153. Furthermore, beacon related information 156 is stored in the storing unit 153.

The program 154 is, for example, an operating system and is positioned between a user or an application program and hardware. The program 154 provides a standard interface to the user or the application program and, at the same time, manages resources such as the hardware efficiently.

The application 155 causes the server 100 to receive the identification information transmitted from the user terminal 50. The application 155 causes the server 100 to acquire the beacon ID and the user ID that are included in the received identification information. The application 155 causes the server 100 to acquire the address of an information processing device ID and the reception location information that are associated with the acquired beacon ID from the beacon related information 156 in the storing unit 153. The application 155 causes the server 100 to create a location information notification which includes the acquired user ID and reception location information and which is addressed to the address of the information processing device 200. The application 155 causes the server 100 to transmit the created location information notification to the information processing device 200.

The application 155 causes the server 100 to receive the provision information notification transmitted from the information processing device 200. The application 155 causes the server 100 to acquire the provision information and the user ID that are included in the received provision information notification. The application 155 causes the server 100 to create user notification information which includes the acquired provision information and which is addressed to the user terminal 50 corresponding to the user ID. The application 155 causes the server 100 to transmit the created user notification information to the user terminal 50.

### (Beacon Related Information)

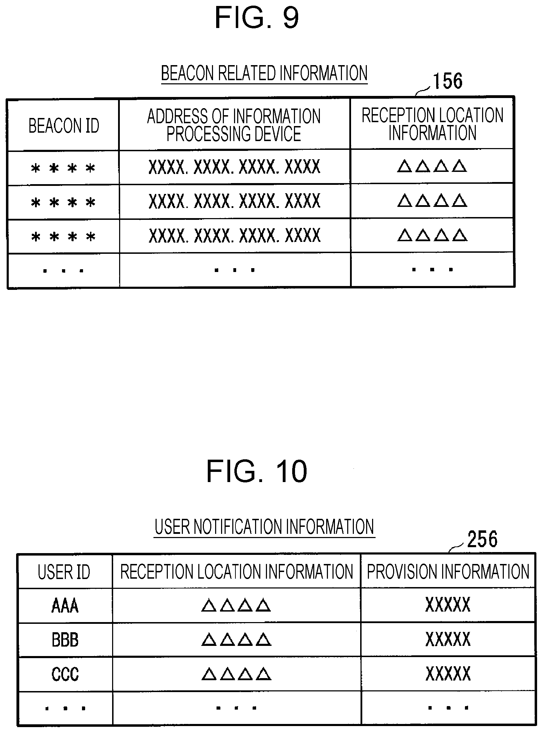

Fig. 9 is a diagram illustrating an example of beacon related information.

For example, the beacon related information 156 is information in a table format in which a beacon ID, the address of an information processing device, and reception location information are associated with one another. In the example illustrated in Fig. 9, a beacon ID "^{∗∗∗∗}", the address of an information processing device "xxxx.xxxx.xxxx.xxxx", and reception location information "ΔΔΔΔ" are stored in association with one another. Explanation will be continued below with reference back to Fig. 8.

The entire or part of the information processing unit 157 is a software functional unit that is implemented when, for example, a processor such as a CPU executes the program 154 and the application 155 stored in the storing unit 153. The entire or part of the information processing unit 157 may be implemented by hardware such as an LSI, an ASIC, or an FPGA or may be implemented by a combination of the software functional unit and the hardware.

The information processing unit 157 includes, for example, a reception part 158 and a creation part 159.

The reception part 158 acquires the identification information output from the communication unit 151, and receives the acquired identification information. The reception part 158 outputs the received identification information to the creation part 159. The reception part 158 acquires the provision information notification output from the communication unit 151, and receives the acquired provision information notification. The reception part 158 outputs the received provision information notification to the creation part 159.

The creation part 159 acquires the identification information output from the reception part 158, and acquires the beacon ID and the user ID that are included in the acquired identification information. The creation part 159 acquires the address of the information processing device 200 that is associated with the acquired beacon ID and the reception location information from the beacon related information 156 stored in the storing unit 153. The creation part 159 creates a location information notification which includes the acquired user ID and reception location information and which is addressed to the address of the information processing device 200. The creation part 159 outputs the created location information notification to the communication unit 151.

The creation part 159 acquires the provision information notification output from the reception part 158, and acquires the provision information and the user ID that are included in the acquired provision information notification. The creation part 159 creates a user notification information which includes the acquired provision information and which is addressed to the address of the user terminal 50 corresponding to the user ID. The creation part 159 outputs the created user notification information to the communication unit 151.

### (Information Processing Device)

The information processing device 200 is implemented by a device such as a personal computer or an industrial computer. However, the information processing device 200 is not limited to the examples mentioned above. The information processing device 200 includes a communication unit 251, a memory 252, a storing unit 253, an information processing unit 257, and a bus line 263 such as an address bus or a data bus for electrically connecting the components of the information processing device 200 as illustrated in Fig. 8.

The communication unit 251 is implemented by a communication module. Specifically, the communication unit 251 is configured to be a device that performs wired communication. Furthermore, the communication unit 251 may be configured to be a wireless device that performs wireless communication using a wireless communication technique such as a wireless LAN (registered trademark). The communication unit 251 communicates with an external communication device such as the server 100 via the communication network NET. Specifically, the communication unit 251 receives a location information notification transmitted from the server 100, and outputs the received location information notification to the information processing unit 257. The communication unit 251 acquires a provision information notification output from the information processing unit 257, and transmits the acquired provision information notification to the server 100.

The memory 252 includes a RAM such as a volatile memory including a semiconductor element and is used as a work memory for the information processing unit 257. The memory 252 includes, for example, a flash memory or the like.

The storing unit 253 is implemented by, for example, a RAM, a ROM, an HDD, a flash memory, a hybrid storage device including a combination of some of the RAM, the ROM, the HDD, and the flash memory, or the like. A program 254 and an application 255 that are to be executed by the information processing unit 257 are stored in the storing unit 253. Furthermore, user notification information 256 is stored in the storing unit 253.

The program 254 is, for example, an operating system and is positioned between a user or an application program and hardware. The program 254 provides a standard interface to the user or the application program and, at the same time, manages resources such as the hardware efficiently.

The application 255 causes the information processing device 200 to receive the location information notification transmitted from the server 100. The application 255 causes the information processing device 200 to acquire the user ID and the reception location information included in the received location information notification. The application 255 causes the information processing device 200 to acquire provision information that is stored in association with the combination of the acquired user ID and reception location information from the user notification information 256 stored in the storing unit 253. The application 255 causes the information processing device 200 to create a provision information notification which includes the acquired provision information and user ID and which is addressed to the address of the server 100. The application 255 causes the information processing device 200 to transmit the created provision information notification to the server 100.

### (User Notification Information)

Fig. 10 is a diagram illustrating an example of user notification information.

For example, the user notification information 256 is information in a table format in which a user ID, reception location information, and provision information are associated with one another. In the example illustrated in Fig. 10, a user ID "AAA", reception location information "ΔΔΔΔ", and provision information "XXXXX" are stored in association with one another, a user ID "BBB", reception location information "ΔΔΔΔ", and provision information "XXXXX" are stored in association with one another, and a user ID "CCC", reception location information "ΔΔΔΔ", and provision information "XXXXX" are stored in association with one another. Explanation will be continued below with reference back to Fig. 8.

The entire or part of the information processing unit 257 is a software functional unit that is implemented when, for example, a processor such as a CPU executes the program 254 and the application 255 stored in the storing unit 253. The entire or part of the information processing unit 257 may be implemented by hardware such as an LSI, an ASIC, or an FPGA or may be implemented by a combination of the software functional unit and the hardware.

The information processing unit 257 includes, for example, a reception part 258 and a creation part 259.

The reception part 258 acquires the location information notification output from the communication unit 251, and receives the acquired location information notification. The reception part 258 outputs the received location information notification to the creation part 259.

The creation part 259 acquires the location information notification output from the reception part 258, and acquires the user ID and the reception location information that are included in the acquired location information notification. The creation part 259 acquires provision information that is stored in association with the combination of the acquired user ID and reception location information from the user notification information 256 stored in the storing unit 253. The creation part 259 creates a provision information notification which includes the acquired provision information and user ID and which is addressed to the address of the server 100. The creation part 259 outputs the created provision information notification to the communication unit 251.

### (Operation of Information Provision System)

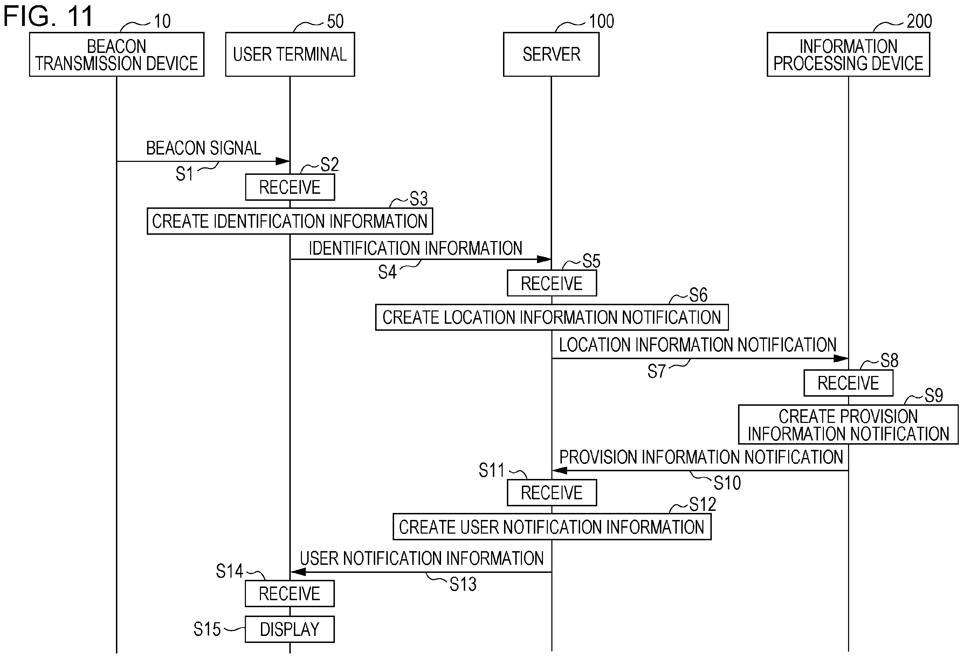

Fig. 11 is a sequence chart illustrating an example of an operation of the information provision system according to the first embodiment.

### (Step S1)

The beacon transmission device 10 transmits a beacon signal.

### (Step S2)

The communication unit 51 of the user terminal 50 receives the beacon signal transmitted from the beacon transmission device 10, and outputs the received beacon signal to the information processing unit 57.

### (Step S3)

The reception part 58 of the information processing unit 57 acquires the beacon signal output from the communication unit 51, and receives the acquired beacon signal The reception part 58 outputs the received beacon signal to the creation part 59.

The creation part 59 acquires the beacon signal output from the reception part 58, and acquires a beacon ID included in the acquired beacon signal. The creation part also acquires a user ID uid stored in the storing unit 53, and creates identification information which includes the acquired user ID uid and beacon ID and which is addressed to the address of the server 100 that is associated with the beacon ID. The creation part 59 outputs the created identification information to the communication unit 51.

### (Step S4)

The communication unit 51 acquires the identification information output from the creation part 59, and transmits the acquired identification information to the server 100.

### (Step S5)

The communication unit 151 of the server 100 receives the identification information transmitted from the user terminal 50, and outputs the received identification information to the information processing unit 157.

### (Step S6)

The reception part 158 of the information processing unit 157 acquires the identification information output from the communication unit 151, and receives the acquired identification information. The reception part outputs the received identification information to the creation part 159.

The creation part 159 acquires the identification information output from the reception part 158, and acquires the beacon ID and the user ID that are included in the acquired identification information. The creation part 159 acquires the address of the information processing device 200 that is associated with the acquired beacon ID and reception location information from the beacon related information 156 stored in the storing unit 153. The creation part 159 creates a location information notification which includes the acquired user ID and reception location information and which is addressed to the address of the information processing device 200. The creation part 159 outputs the created location information notification to the communication unit 151.

### (Step S7)

The communication unit 151 acquires the location information notification output from the creation part 159, and transmits the acquired location information notification to the information processing device 200.

### (Step S8)

The communication unit 251 of the information processing device 200 receives the location information notification transmitted from the server 100, and outputs the received location information notification to the information processing unit 257.

### (Step S9)

The reception part 258 of the information processing unit 257 acquires the location information notification output from the communication unit 251, and receives the acquired location information notification. The reception part 258 outputs the received location information notification to the creation part 259.

The creation part 259 acquires the location information notification output from the reception part 258, and acquires the user ID and the reception location information that are included in the acquired location information notification. The creation part 259 acquires provision information that is stored in association with the combination of the acquired user ID and reception location information from the user notification information 256 stored in the storing unit 253. The creation part 259 creates a provision information notification which includes the acquired provision information and user ID and which is addressed to the address of the server 100. The creation part 259 outputs the created provision information notification to the communication unit 251.

### (Step S10)

The communication unit 251 acquires the provision information notification output from the creation part 259, and transmits the acquired provision information notification to the server 100.

### (Step S11)

The communication unit 151 of the server 100 receives the provision information notification transmitted from the information processing device 200, and outputs the received provision information notification to the information processing unit 157.

### (Step S12)

The reception part 158 of the information processing unit 157 acquires the provision information notification output from the communication unit 151, and receives the acquired provision information notification. The reception part 158 outputs the received provision information notification to the creation part 159.

The creation part 159 acquires the provision information notification output from the reception part 158, and acquires the provision information and the user ID that are included in the acquired provision information notification. The creation part 159 creates user notification information which includes the acquired provision information and which is addressed to the address of the user terminal 50 corresponding to the user ID. The creation part 159 outputs the created user notification information to the communication unit 151.

### (Step S13)

The communication unit 151 acquires the user notification information output from the creation part 159, and transmits the acquired user notification information to the user terminal 50.

### (Step S14)

The communication unit 51 of the user terminal 50 receives the user notification information transmitted from the server 100, and outputs the received user notification information to the information processing unit 57.

### (Step S15)

The reception part 58 of the information processing unit 57 acquires the user notification information output from the communication unit 51, and receives the acquired user notification information. The reception part 58 outputs the received user notification information to the processing part 60.

The processing part 60 acquires the user notification information output from the reception part 58, processes the acquired user notification information, and displays a result of the processing on the display unit 62.

In the first embodiment described above, the case where the beacon transmission device 10 is installed in a smoking area has been described. However, the present invention is not limited to the example described above. For example, the beacon transmission device 10 may be installed in a desired place such as a store.

In the first embodiment described above, the case where a single beacon transmission device 10, a single user terminal 50, a single server 100, and a single information processing device 200 are provided has been described. However, the present invention is not limited to the example described above. For example, two or more beacon transmission devices 10, two or more user terminals 50, two or more servers 100, and two or more information processing devices 200 may be provided.

In the first embodiment described above, the case where the information processing device 200 acquires provision information that is stored in association with the combination of a user ID and reception location information has been described. However, the present invention is not limited to the example described above. For example, the information processing device 200 may acquire provision information that is stored in association with one of a user ID and reception location information.

In the first embodiment described above, the case where the information provision system includes the server 100 and the information processing device 200 has been described. However, the present invention is not limited to the example described above. For example, a function of the server 100 may be executed by the information processing device 200.

In the first embodiment described above, at the timing when either one of or both the case where the same beacon ID continues to be acquired for a predetermined period of time and the case where the same beacon ID is received a predetermined number of times or more during the predetermined period of time is satisfied, the creation part 59 of the user terminal 50 may acquire the user ID uid stored in the storing unit 53 and create identification information which includes the acquired user ID uid and beacon ID and which is addressed to the address of the server 100 that is associated with the beacon ID. The predetermined period of time may be any time such as ten seconds, one minute, or five minutes. Furthermore, the predetermined number of times may be any number of times such as five times or ten times. With the arrangement described above, no identification information is transmitted from the user terminal 50 that is held by a user who only passes in the vicinity of the smoking area in which the beacon transmission device 10 is installed. That is, transmission of identification information from the user terminal 50 held by a user who only passes in the vicinity of the smoking area in which the beacon transmission device 10 is installed can be prevented. Thus, information can be provided to a user who is in the smoking area in which the beacon transmission device 10 is installed.

In the first embodiment described above, at the timing when either one of or both the case where the same beacon ID and the same user ID continue to be acquired for a predetermined period of time and the case where the same beacon ID and the same user ID are received a predetermined number of times or more during the predetermined period of time is satisfied, the creation part 159 of the server 100 may acquire the address of an information processing device ID and reception location information that are associated with the acquired beacon ID from the beacon related information 156 stored in the storing unit 153. The predetermined period of time may be any time such as ten seconds, one minute, or five minutes. Furthermore, the predetermined number of times may be any number of times such as five times or ten times. With the arrangement described above, no location information notification that is triggered by identification information transmitted from the user terminal 50 held by a user who only passes in the vicinity of the smoking area in which the beacon transmission device 10 is installed is transmitted from the server 100. That is, transmission of a location information notification from the server 100 can be prevented even in the case where the user terminal 50 held by a user who only passes in the vicinity of the smoking area in which the beacon transmission device 10 is installed transmits identification information. Thus, information can be provided to a user who is in the smoking area in which the beacon transmission device 10 is installed.

With the information provision system 1 according to the first embodiment, the information processing device 200 is capable of transmitting provision information associated with the user ID of the user of the aerosol generation device 70 and the reception location information to the user terminal 50 held by the user. Provision information can be set according to the user of the aerosol generation device 70. Thus, the variety of information to be provided to the user terminal 50 that receives a beacon signal can be increased.

### (Modification)

An information provision system 1a according to a modification of the first embodiment is different from the information provision system 1 according to the first embodiment in that the information provision system 1a provides information pertaining to a reward to the user of the aerosol generation device 70. In this modification, as an example of a reward, a case where a point or points are granted on the basis of the number of times that a location information notification has been received will be described below.

Fig. 1 may be applied to the information provision system 1a according to the modification of the first embodiment. However, the information provision system 1a is different from the information provision system 1 according to the first embodiment in that the information provision system 1a includes an information processing device 200a in place of the information processing device 200.

### (Information Processing Device)

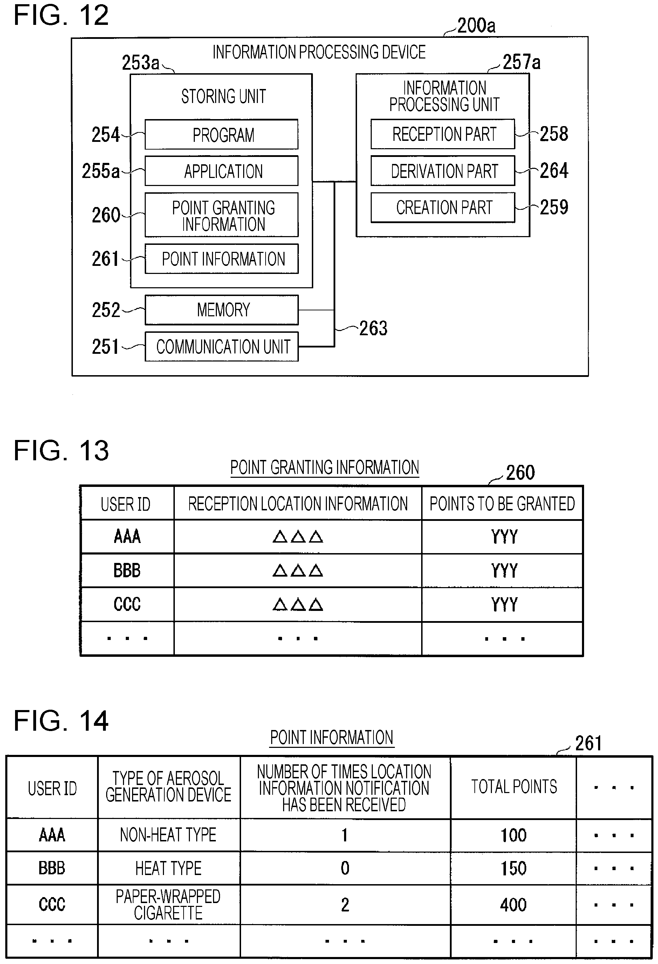

Fig. 12 is a block diagram illustrating an example of an information processing device according to a modification of the first embodiment.

The information processing device 200a is implemented by a device such as a personal computer or an industrial computer. However, the information processing device 200a is not limited to the examples mentioned above. The information processing device 200a includes a communication unit 251, a memory 252, a storing unit 253a, an information processing unit 257a, and a bus line 263 such as an address bus or a data bus for electrically connecting the components of the information processing device 200a as illustrated in Fig. 12.

The storing unit 253a is implemented by, for example, a RAM, a ROM, an HDD, a flash memory, a hybrid storage device including a combination of some of the RAM, the ROM, the HDD, and the flash memory, or the like. The program 254 and an application 255a that are to be executed by the information processing unit 257a are stored in the storing unit 253a. Furthermore, point granting information 260 and point information 261 are stored in the storing unit 253a.

The application 255a causes the information processing device 200a to execute functions described below, in addition to functions similar to the functions that the application 255 causes the information processing device 200 to execute in the first embodiment. The application 255a causes the information processing device 200a to acquire the user ID and the reception location information that are included in the received location information notification and then acquire a point or points to be granted that are stored in association with the combination of the acquired user ID and reception location information from the point granting information 260 stored in the storing unit 253a. The application 255a causes the information processing device 200a to derive an updated point or points by adding the acquired granted point or points to the total points stored in association with the user ID from the point information 261 stored in the storing unit 253a. The application 255a causes the information processing device 200a to update the total points, which are stored in association with the user ID, with the derived updated point or points. The application 255a causes the information processing device 200a to acquire the total points stored in association with the user ID from the point information 261 stored in the storing unit 253a. The application 255a causes the information processing device 200a to create a provision information notification which includes the acquired total points and user ID and which is addressed to the address of the server 100. The application 255a causes the information processing device 200a to transmit the created provision information notification to the server 100.

### (Point Granting Information)

Fig. 13 is a diagram illustrating an example of point granting information.

For example, the point granting information 260 is information in a table format in which a user ID, reception location information, and a point or points to be granted are associated with one another. In the example illustrated in Fig. 13, a user ID "AAA", reception location information "ΔΔΔΔ", and points to be granted "YYY" are stored in association with one another, a user ID "BBB", reception location information "ΔΔΔΔ", and points to be granted "YYY" are stored in association with one another, and a user ID "CCC", reception location information "ΔΔΔΔ", and points to be granted "YYY" are stored in association with one another.

For example, in the point granting information 260, points to be granted may be a negative value based on reception location information. For example, in the case where the reception location information represents a smoking area, points to be granted may be a positive value. In the case where the reception location information represents a place other than a smoking area, points to be granted may be a negative value. With this arrangement, the user can be prompted to smoke in a smoking area.

### (Point Information)

Fig. 14 is a diagram illustrating an example of point information.

For example, the point information 261 is information in a table format in which a user ID, information indicating the type of an aerosol generation device owned by the user corresponding to the user ID, information indicating the number of times that a location information notification has been received, and information indicating total points are associated with one another. In the example illustrated in Fig. 14, a user ID "AAA", the type of an aerosol generation device "non-heat type", the number of times that a location information notification has been received "1", and total points "100" are stored in association with one another, a user ID "BBB", the type of an aerosol generation device "heat type", the number of times that a location information notification has been received "0", and total points "150" are stored in association with one another, and a user ID "CCC", the type of an aerosol generation device "paper-wrapped cigarette", the number of times that a location information notification has been received "2", and total points "400" are stored in association with one another. Explanation will be continued below with reference back to Fig. 12.

The entire or part of the information processing unit 257a is a software functional unit that is implemented when, for example, a processor such as a CPU executes the program 254 and the application 255a stored in the storing unit 253a. The entire or part of the information processing unit 257a may be implemented by hardware such as an LSI, an ASIC, or an FPGA or may be implemented by a combination of the software functional unit and the hardware.

The information processing unit 257a includes, for example, a reception part 258, a derivation part 264, and a creation part 259a.

The reception part 258 outputs the received location information notification to the derivation part 264 and the creation part 259.

The derivation part 264 acquires the location information notification output from the reception part 258, and acquires the user ID and the reception location information that are included in the acquired location information notification. The derivation part 264 acquires a point or points to be granted that are stored in association with the combination of the acquired user ID and reception location information from the point granting information 260 stored in the storing unit 253a. The derivation part 264 derives, in accordance with the user ID, updated points by adding the acquired granted point or points to the total points that are stored in association with the user ID from the point information 261 stored in the storing unit 253a. The derivation part 264 updates the total points, which are stored in association with the user ID, with the derived updated points.

The creation part 259a acquires the location information notification output from the reception part 258, and acquires the user ID and the reception location information that are included in the acquired location information notification. The creation part 259a acquires information indicating the total points stored in association with the acquired user ID from the point information 261 stored in the storing unit 253a. The creation part 259a creates a provision information notification which includes the acquired information indicating the total points and the user ID and which is addressed to the address of the server 100. The creation part 259a outputs the created provision information notification to the communication unit 251.

Fig. 11 may be applied to an operation of the information provision system 1a according to the first embodiment. However, the operation of the information provision system 1a is different from the operation illustrated in Fig. 11 in the processing of step S9.

In step S9, the reception part 258 of the information processing unit 257a acquires the location information notification output from the communication unit 251, and receives the acquired location information notification. The reception part 258 outputs the received location information notification to the derivation part 264 and the creation part 259.

The derivation part 264 acquires the location information notification output from the reception part 258, and acquires the user ID and the reception location information that are included in the acquired location information notification. The derivation part 264 acquires a point or points to be granted that are stored in association with the combination of the acquired user ID and reception location information from the point granting information 260 stored in the storing unit 253a. The derivation part 264 derives, in accordance with the user ID, updated points by adding the acquired granted point or points to the total points stored in association with the user ID from the point information 261 stored in the storing unit 253a. The derivation part 264 updates the total points, which are stored in association with the user ID, with the derived updated points.

The creation part 259a acquires the location information notification output from the reception part 258, and acquires the user ID and the reception location information that are included in the acquired location information notification. The creation part 259a acquires information indicating the total points stored in association with the acquired user ID from the point information 261 stored in the storing unit 253a. The creation part 259a creates a provision information notification which includes the acquired information indicating the total points and the user ID and which is addressed to the address of the server 100. The creation part 259a outputs the created provision information notification to the communication unit 251.

In the modification of the first embodiment, the user notification information 256 may be stored in the storing unit 253a of the information processing device 200a, so that the creation part 259a acquires provision information stored in association with the combination of the acquired user ID and reception location information from the user notification information 256 stored in the storing unit 253a. The creation part 259a may create a provision information notification which includes the information indicating the total points, the provision information, and the user ID and which is addressed to the address of the server 100.

With the information provision system 1a according to the modification of the first embodiment, the information processing device 200a is capable of transmitting information indicating the total points associated with the user ID of the user of the aerosol generation device 70 to the user terminal 50 held by the user of the aerosol generation device 70. The information indicating the total points granted to the user can be provided to the user of the aerosol generation device 70. Thus, the variety of information to be provided to the user terminal 50 that receives a beacon signal can be increased.

### (Second Embodiment)

### (Information Provision System)

An information provision system according to a second embodiment of the present invention will be described with reference to drawings.

Fig. 1 may be applied to an example of an information provision system 1b according to the second embodiment.

The information provision system 1b is different from the information provision system 1 according to the first embodiment in that the beacon transmission device 10 transmits a beacon signal when the beacon transmission device 10 detects the aerosol generation device 70.

Hereinafter, the aerosol generation device 70 and the beacon transmission device 10 that are included in the information provision system 1b will be described.

### (Aerosol Generation Device)

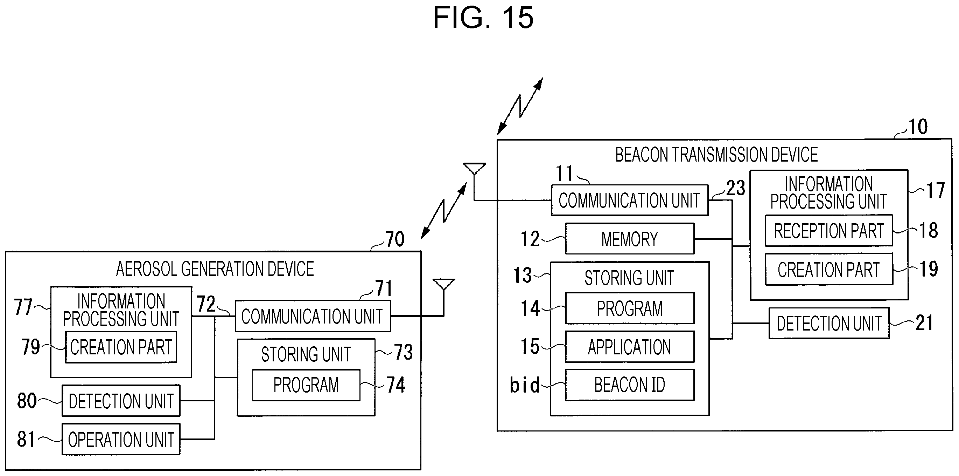

Fig. 15 is a block diagram illustrating an example of an aerosol generation device and a beacon transmission device that are included in the information provision system according to the second embodiment.

The aerosol generation device 70 includes a communication unit 71, a storing unit 73, an information processing unit 77, a detection unit 80, an operation unit 81, and a bus line 72 such as an address bus or a data bus for electrically connecting the components of the aerosol generation device 70 as illustrated in Fig. 15. The schematic configuration diagram of the aerosol generation device 70 according to the second embodiment is similar to those of the aerosol generation device 70 according to the first embodiment illustrated in Figs. 2 to 7.

The communication unit 71 is implemented by a communication module. Specifically, the communication unit 71 is configured to be a wireless device that performs wireless communication using a wireless communication technique such as a wireless LAN (registered trademark) or Bluetooth (registered trademark). The communication unit 71 transmits a wireless signal such as a beacon signal.

The storing unit 73 is implemented by, for example, a RAM, a ROM, a flash memory, a hybrid storage device including a combination of some of the RAM, the ROM, and the flash memory, or the like. A program 74 to be executed by the information processing unit 77 is stored in the storing unit 73.

When a predetermined operation, such as pressing on a button included in the operation unit 81, is performed, the program 74 causes the aerosol generation device 70 to create a beacon signal including a beacon ID and transmit the created beacon signal. Furthermore, when a predetermined operation, such as pressing on a button included in the operation unit 81, is performed, the program 74 may cause the aerosol generation device 70 to create a beacon signal including location information of the aerosol generation device 70 and a beacon ID and transmit the created beacon signal. In this example, a case where, when a predetermined operation, such as pressing on a button included in the operation unit 81, is performed, the program 74 causes the aerosol generation device 70 to create a beacon signal including a beacon ID and transmit the created beacon signal, will be described below.

The detection unit 80 detects the location of the aerosol generation device 70, and outputs location information of the detected aerosol generation device 70 to the information processing unit 77. For example, the detection unit 80 includes a positioning system such as a global positioning system (GPS).

The operation unit 81 includes, for example, a button. When the operation unit 81 detects that the button is pressed, the operation unit 81 outputs information indicating pressing on the button to the information processing unit 77.

The entire or part of the information processing unit 77 is a software functional unit that is implemented when, for example, a processor such as a CPU executes the program 74 stored in the storing unit 73. The entire or part of the information processing unit 77 may be implemented by hardware such as an LSI, an ASIC, or an FPGA or may be implemented by a combination of the software functional unit and the hardware.

The information processing unit 77 includes, for example, a creation part 79.

When information output from the operation unit 81 indicating that the button is pressed is acquired, the creation part 79 creates a beacon signal including the ID of the aerosol generation device 70, and outputs the created beacon signal to the communication unit 71.

### (Beacon Transmission Device)

The beacon transmission device 10 includes a communication unit 11, a memory 12, a storing unit 13, an information processing unit 17, a detection unit 21, and a bus line 23 such as an address bus or a data bus for electrically connecting the components of the beacon transmission device 10 as illustrated in Fig. 15.

The communication unit 11 is implemented by a communication module. Specifically, the communication unit 11 is configured to be a wireless device that performs wireless communication using a wireless communication technique such as a wireless LAN (registered trademark) or Bluetooth (registered trademark). The communication unit 11 receives a beacon signal transmitted from the aerosol generation device 70, and outputs the received beacon signal to the information processing unit 17. The communication unit 11 outputs the beacon signal output from the information processing unit 17 to the communication unit 11.

The memory 12 includes a RAM such as a volatile memory including a semiconductor element and is used as a work memory for the information processing unit 17. The memory 12 includes, for example, a flash memory or the like.

The storing unit 13 is implemented by, for example, a RAM, a ROM, an HDD, a flash memory, a hybrid storage device including a combination of some of the RAM, the ROM, the HDD, and the flash memory, or the like. A program 14 and an application 15 that are to be executed by the information processing unit 17 are stored in the storing unit 13. Furthermore, a beacon ID bid is stored in the storing unit 13.

The program 14 is, for example, an operating system and is positioned between a user or an application program and hardware. The program 14 provides a standard interface to the user or the application program and, at the same time, manages resources such as the hardware efficiently.

The application 15 causes the beacon transmission device 10 to receive a beacon signal transmitted from the aerosol generation device 70. Reception of the beacon signal by the beacon transmission device 10 triggers the application 15 to cause the beacon transmission device 10 to acquire the beacon ID bid stored in the storing unit 13 and create a beacon signal including the acquired beacon ID bid.

Furthermore, the application 15 causes the beacon transmission device 10 to detect the aerosol generation device 70. Detection of the aerosol generation device 70 by the beacon transmission device 10 triggers the application 15 to cause the beacon transmission device 10 to acquire the beacon ID bid stored in the storing unit 13 and generate a beacon signal including the acquired beacon ID bid. The application 15 causes the beacon transmission device 10 to transmit the generated beacon signal.

The beacon ID bid is information for identifying the beacon signal transmitted from the beacon transmission device 10.

The entire or part of the information processing unit 17 is a software functional unit that is implemented when, for example, a processor such as a CPU executes the program 14 and the application 15 that are stored in the storing unit 13. The entire or part of the information processing unit 17 may be implemented by hardware such as an LSI, an ASIC, or an FPGA or may be implemented by a combination of the software functional unit and the hardware.

The information processing unit 17 includes, for example, a reception part 18 and a creation part 19.

The reception part 18 acquires the beacon signal output from the communication unit 11, and receives the acquired beacon signal. The reception part 18 outputs the received beacon signal to the creation part 19. Furthermore, the reception part 18 acquires information output from the detection unit 21 indicating that the detection unit 21 has detected the aerosol generation device 70, and outputs the acquired information indicating that the aerosol generation device 70 has been detected to the creation part 19.

Acquisition of one or both of the beacon signal and the information indicating that the aerosol generation device 70 has been detected that are output from the reception part 18 triggers the creation part 19 to acquire the beacon ID bid stored in the storing unit 13. The creation part 19 creates a beacon signal including the acquired beacon ID bid. The creation part 19 outputs the created beacon signal to the communication unit 11.

The detection unit 21 detects the aerosol generation device 70. Specifically, the detection unit 21 includes an image sensor. The detection unit 21 stores image characteristics of an aerosol generation device. The detection unit 21 extracts image characteristics of an object from an image captured by an imaging device and determines, based on the extracted image characteristics of the object and the stored image characteristics of the aerosol generation device, whether or not the image captured by the imaging device represents the aerosol generation device. In the case where it is determined that the aerosol generation device is imaged, the detection unit 21 outputs information indicating that the aerosol generation device 70 has been detected to the information processing unit 17.

### (Operation of Information Provision System)

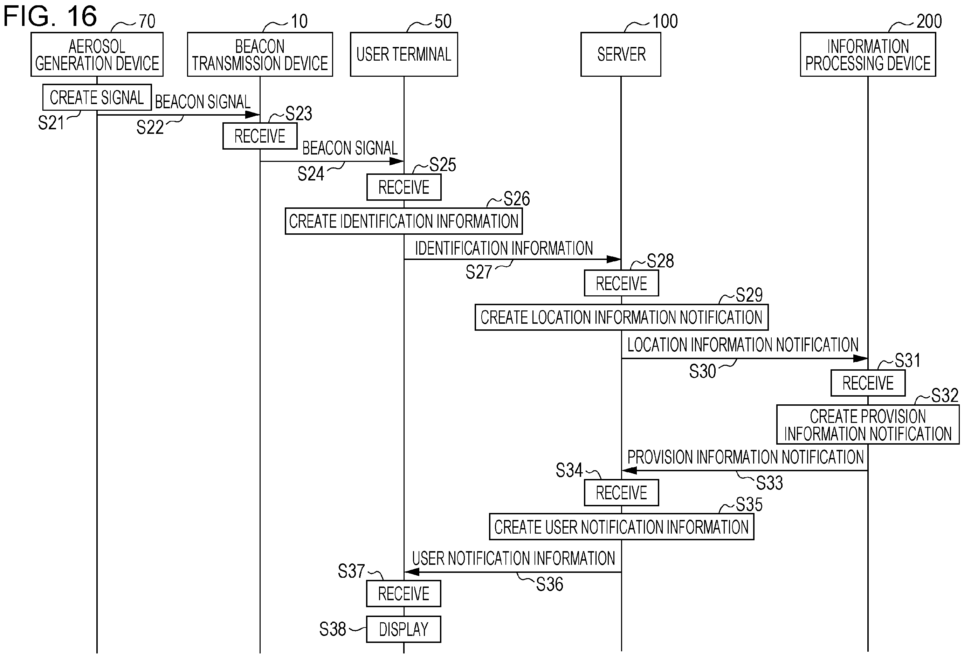

Fig. 16 is a sequence chart illustrating an example of an operation of the information provision system according to the second embodiment. A case where, when the beacon transmission device 10 receives a beacon signal transmitted from the aerosol generation device 70, the beacon transmission device 10 transmits a beacon signal to be received by the user terminal 50 will be described with reference to Fig. 16.

### (Step S21)

When the creation part 79 of the aerosol generation device 70 acquires information output from the operation unit 81 indicating that the button is pressed, the creation part 79 of the aerosol generation device 70 creates a beacon signal including a beacon ID.

### (Step S22)

The creation part 79 of the aerosol generation device 70 outputs the created beacon signal to the communication unit 71. The communication unit 71 acquires the beacon signal output from the creation part 79, and transmits the acquired beacon signal.

### (Step S23)

The communication unit 11 of the beacon transmission device 10 receives the beacon signal transmitted from the aerosol generation device 70, and outputs the received beacon signal to the information processing unit 17. The reception part 18 acquires the beacon signal output from the communication unit 11, and receives the acquired beacon signal. The reception part 18 outputs the received beacon signal to the creation part 19. Acquisition of the beacon signal output from the reception part 18 triggers the creation part 19 to acquire a beacon ID bid stored in the storing unit 13. The creation part 19 creates a beacon signal including the acquired beacon ID bid.

### (Step S24)

The creation part 19 outputs the created beacon signal to the communication unit 11. The communication unit 11 acquires the beacon signal output from the creation part 19, and transmits the acquired beacon signal.

Steps S2 to S15 described above in Fig. 11 may be applied to steps S25 to S38.

In the second embodiment described above, the case where the beacon transmission device 10 transmits a beacon signal to be received by the user terminal 50 when either one of or both the case where a beacon signal transmitted from the aerosol generation device 70 is received and the case where the beacon transmission device 10 has detected the aerosol generation device 70 is satisfied, has been described. However, the present invention is not limited to the example mentioned above. For example, in the case where communication is established between the aerosol generation device 70 and the beacon transmission device 10 by using a short-range wireless communication technique such as FeliCa (registered trademark), the beacon transmission device 10 may transmit a beacon signal. In the second embodiment described above, the case where the aerosol generation device 70 creates a beacon signal including a beacon ID and transmits the created beacon signal when a predetermined operation such as pressing on the button included in the operation unit 81 is performed, has been described. However, the present invention is not limited to the example mentioned above. For example, when a predetermined operation such as pressing on the button included in the operation unit 81 is performed, the aerosol generation device 70 may create a beacon signal including location information of the aerosol generation device 70 and a beacon ID and transmit the created beacon signal In this case, the user terminal 50 receives the beacon signal transmitted from the aerosol generation device 70, acquires the beacon ID and the location information of the aerosol generation device 70 that are included in the received beacon signal, creates identification information including the acquired beacon ID, location information of the aerosol generation device 70, and a user ID uid, and transmits the created identification information to the server 100. The subsequent processing is performed based on the location information of the aerosol generation device 70 in place of the reception location information. With this arrangement, even in an environment in which the beacon transmission device 10 is not installed, information can be provided to the user terminal held by the user of the aerosol generation device 70. With the information provision system according to the second embodiment, when detecting the aerosol generation device 70, the beacon transmission device 10 transmits a beacon signal. Thus, power saving of the beacon transmission device 10 can be achieved, and information can be provided to a user who is in a smoking area in which the beacon transmission device 10 is installed.

### (Third Embodiment)

### (Information Provision System)

An information provision system according to a third embodiment of the present invention will be described with reference to drawings. When the user of the aerosol generation device 70 has lost the aerosol generation device 70, an information provision system 1c according to the third embodiment notifies the user terminal 50 held by the user of the location of the missing aerosol generation device 70.

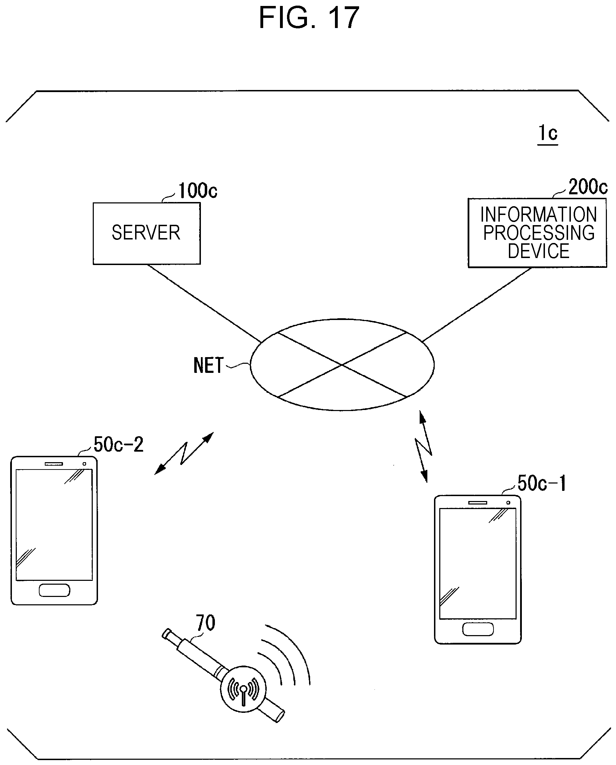

Fig. 17 is a diagram illustrating an example of the information provision system according to the third embodiment. The information provision system 1c according to this embodiment includes a user terminal 50c-1, a user terminal 50c-2, the aerosol generation device 70, a server 100c, and an information processing device 200c. The user terminal 50c-1, the user terminal 50c-2, the server 100c, and the information processing device 200c are connected to one another via the communication network NET.

The user holds the aerosol generation device 70 and the user terminal 50c-2. The aerosol generation device 70 and the user terminal 50c-2 communicate with each other.

Disconnection of the pairing between the aerosol generation device 70 and the user terminal 50c-2 triggers the aerosol generation device 70 to transmit a beacon signal including the ID of the aerosol generation device 70 and a beacon ID.

The user terminal 50c-1 stores the beacon ID and the address of the server 100c in association with each other. The user terminal 50c-1 receives the beacon signal transmitted from the aerosol generation device 70, and creates identification information which includes the ID of the aerosol generation device 70 and the beacon ID that are included in the received beacon signal and which is addressed to the address of the server 100c associated with the beacon ID. The user terminal 50c-1 transmits the created identification information to the server 100c. The ID of the aerosol generation device 70 is an identifier that can uniquely identify the aerosol generation device.

The server 100c is included in a message exchange system capable of transmitting a message to the user terminal 50c-1 and the user terminal 50c-2. The server 100c stores a beacon ID, the address of the information processing device 200c, and reception location information in association with one another. The server 100c receives the identification information transmitted from the user terminal 50c-1, and acquires the ID of the aerosol generation device 70 and the beacon ID that are included in the received identification information. The server 100c acquires the address of the information processing device 200c and the reception location information that are associated with the acquired beacon ID, creates a location information notification which includes the ID of the aerosol generation device 70 and the reception location information and which is addressed to the address of the information processing device 200c, and transmits the created location information notification to the information processing device 200c.

The information processing device 200c receives the location information notification transmitted from the server 100c, and acquires the ID of the aerosol generation device 70 and the reception location information that are included in the received location information notification. The information processing device 200c creates location information in which the acquired ID of the aerosol generation device 70 and reception location information are associated with each other, and stores the created location information.

The user terminal 50c-2 stores the beacon ID and the address of the server 100c in association with each other. The user terminal 50c-2 stores the ID of the aerosol generation device 70, the beacon ID included in the beacon signal transmitted from the aerosol generation device 70, and the ID of the aerosol generation device 70. The user terminal 50c-2 creates a signal (hereinafter, referred to as a "location information request") for requesting location information which includes the ID of the aerosol generation device 70 and which is addressed to the address of the server 100c that is associated with the beacon ID. The user terminal 50c-2 transmits the created location information request to the server 100c.

The server 100c receives the location information request transmitted from the user terminal 50c-2, and acquires the ID of the aerosol generation device 70 included in the received location information request. The server 100c creates a signal (hereinafter, referred to as a "location information acquisition request") for requesting acquisition of location information which includes the acquired ID of the aerosol generation device 70 and which is addressed to the information processing device 200, and transmits the created location information acquisition request to the information processing device 200c.

The information processing device 200c receives the location information acquisition request transmitted from the server 100c, and acquires the ID of the aerosol generation device 70 included in the received location information request. The information processing device 200c acquires the reception location information stored in association with the ID of the aerosol generation device 70, on the basis of the acquired ID of the aerosol generation device 70, from the stored location information. The information processing device 200c creates information (hereinafter, referred to as "location notification information") for notifying the location which includes the acquired reception location information and which is addressed to the server 100c, and transmits the created location notification information to the server 100c.

The server 100c receives the location notification information transmitted from the information processing device 200c, acquires the reception location information included in the received location notification information, and creates location information which includes the acquired reception location information and which is addressed to the user terminal 50c-2, and transmits the created location information to the user terminal 50c-2.

The user terminal 50c-2 receives the location information transmitted from the server 100c, processes the reception location information included in the received location information, and thus displays a result of the processing.

Hereinafter, any one of the user terminal 50c-1 and the user terminal 50c-2 will be described as a user terminal 50c.

Hereinafter, the user terminal 50c, the aerosol generation device 70, the server 100c, and the information processing device 200c that are included in the information provision system 1c will be described.

### (User Terminal)

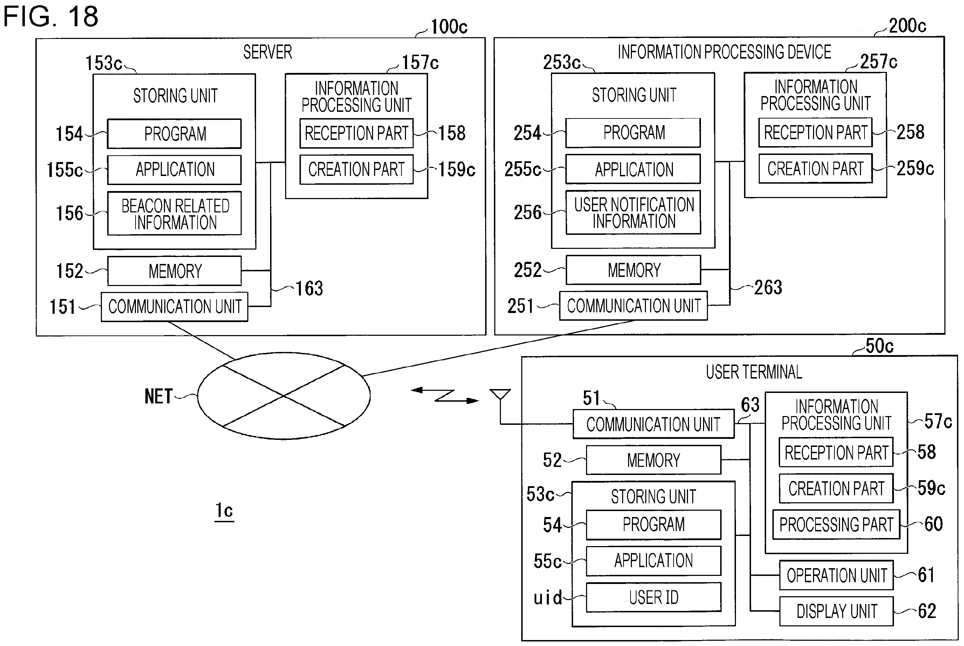

Fig. 18 is a block diagram illustrating an example of the information provision system according to the third embodiment. The user terminal 50c is implemented by a smartphone, a portable terminal, a personal computer, a tablet terminal device, a wristwatch-type terminal device, a glasses-type terminal device, or other types of information processing equipment. Furthermore, the user terminal 50 may be a smart speaker (AI speaker; "AI" is an abbreviation of "artificial intelligence") or a head mount display (HMD: Head Mounted Display). However, the user terminal 50 is not limited to the examples mentioned above. The user terminal 50c includes a communication unit 51, a memory 52, a storing unit 53c, an information processing unit 57c, an operation unit 61, a display unit 62, and a bus line 63 such as an address bus or a data bus for electrically connecting the components of the user terminal 50c as illustrated in Fig. 18.

The communication unit 51 receives location information transmitted from the server 100c, and outputs the received location information to the information processing unit 57c.

The storing unit 53c is implemented by, for example, a RAM, a ROM, an HDD, a flash memory, a hybrid storage device including a combination of some of the RAM, the ROM, the HDD, and the flash memory, or the like. A program 54 and an application 55c that are to be executed by the information processing unit 57c are stored in the storing unit 53c. Furthermore, a user ID uid is stored in the storing unit 53c.

The application 55c causes the user terminal 50c to execute functions described below, in addition to functions similar to the functions that the application 55 causes the user terminal 50c to execute in the first embodiment. The application 55c causes the user terminal 50c to store the beacon ID and the ID of the aerosol generation device 70 that are included in the beacon signal transmitted from the aerosol generation device 70. Disconnection of communication between the user terminal 50c and the aerosol generation device 70 triggers the application 55c to cause the user terminal 50c to create a location information request which includes the ID of the aerosol generation device 70 and which is addressed to the address of the server 100c associated with the beacon ID. The application 55c causes the user terminal 50c to transmit the created location information request to the server 100c. The application 55c causes the user terminal 50c to receive the location information transmitted from the server 100c, process the reception location information included in the received location information, and thus display a result of the processing.

The entire or part of the information processing unit 57c is a software functional unit that is implemented when, for example, a processor such as a CPU executes the program 54 and the application 55c that are stored in the storing unit 53c. The entire or part of the information processing unit 57c may be implemented by hardware such as an LSI, an ASIC, or an FPGA or may be implemented by a combination of the software functional unit and the hardware.

The information processing unit 57c includes, for example, a reception part 58, a creation part 59c, and a processing part 60.

The reception part 58 acquires information output from the operation unit 61 indicating that location information is requested, and receives the acquired information indicating that the location information is requested. The reception part 58 outputs the received information indicating that the location information is requested to the creation part 59c. The reception part 58 acquires the location information output from the communication unit 51, and outputs the acquired location information to the processing part 60.

When the creation part 59c acquires the information output from the reception part 58 indicating that the location information is requested, the creation part 59c acquires the beacon ID and the ID of the aerosol generation device 70 from the storing unit 53c. The creation part 59c creates a location information request which includes the acquired ID of the aerosol generation device 70 and which is addressed to the address of the server 100c associated with the beacon ID. The creation part 59c outputs the created location information request to the communication unit 51.

The processing part 60 acquires the location information output from the reception part 58, processes the acquired location information, and displays a result of processing on the display unit 62.

### (Aerosol Generation Device)

The aerosol generation device 70 according to the second embodiment described above with reference to Fig. 15 may be applied to the aerosol generation device 70. A schematic configuration diagram of the aerosol generation device 70 according to the third embodiment is similar to the aerosol generation device 70 according to the first embodiment illustrated in Figs. 2 to 7.

### (Server)

The server 100c is implemented by a device such as a personal computer or an industrial computer. However, the server 100c is not limited to the examples mentioned above. The server 100c includes a communication unit 151, a memory 152, a storing unit 153c, an information processing unit 157c, and a bus line 163 such as an address bus or a data bus for electrically connecting the components of the server 100c as illustrated in Fig. 18.

The communication unit 151 receives the location information request transmitted from the user terminal 50c-2, and outputs the received location information request to the information processing unit 157c. The communication unit 151 acquires the location information acquisition request output from the information processing unit 157c, and transmits the acquired location information acquisition request to the information processing device 200c. The communication unit 151 receives the location notification information transmitted from the information processing device 200c, and outputs the received location notification information to the information processing unit 157c. The communication unit 151 acquires the location information output from the information processing unit 157c, and transmits the acquired location information to the user terminal 50c.

The storing unit 153c is implemented by, for example, a RAM, a ROM, an HDD, a flash memory, a hybrid storage device including a combination of some of the RAM, the ROM, the HDD, and the flash memory, or the like. A program 154 and an application 155c that are to be executed by the information processing unit 157 are stored in the storing unit 153c. Furthermore, beacon related information 156 is stored in the storing unit 153c.

The application 155c causes the server 100c to execute functions described below, in addition to functions similar to the functions that the application 155 causes the server 100 to execute in the first embodiment. The application 155c causes the server 100c to receive the location information request transmitted from the user terminal 50c-2. The application 155c causes the server 100c to acquire the ID of the aerosol generation device 70 included in the received location information request. The application 155c causes the server 100c to create a location information acquisition request which includes the acquired ID of the aerosol generation device 70 and which is addressed to the address of the information processing device 200c and transmit the created location information acquisition request to the information processing device 200c. The application 155c causes the server 100c to receive the location notification information transmitted from the information processing device 200c and acquire the reception location information included in the received location notification information. The application 155c causes the server 100c to create location information which includes the acquired reception location information and which is addressed to the address of the user terminal 50c-2 and transmit the created location information to the user terminal 50c-2.

The entire or part of the information processing unit 157c is a software functional unit that is implemented when, for example, a processor such as a CPU executes the program 154 and the application 155c that are stored in the storing unit 153c. The entire or part of the information processing unit 157c may be implemented by hardware such as an LSI, an ASIC, or an FPGA or may be implemented by a combination of the software functional unit and the hardware.

The information processing unit 157c includes, for example, a reception part 158 and a creation part 159c.

The reception part 158 acquires the location information request output from the communication unit 151, and receives the acquired location information request. The reception part 158 outputs the received location information request to the creation part 159c. The reception part 158 acquires the location notification information output from the communication unit 151, and receives the acquired location notification information. The reception part 158 outputs the received location notification information to the creation part 159c.

The creation part 159c acquires the location information request output from the reception part 158, and acquires the ID of the aerosol generation device 70 included in the acquired location information request. The creation part 159c creates a location information acquisition request which includes the acquired ID of the aerosol generation device 70 and which is addressed to the information processing device 200c, and outputs the created location information acquisition request to the communication unit 151. The creation part 159c acquires the location notification information output from the reception part 158, and acquires the reception location information included in the acquired location notification information. The creation part 159c creates location information which includes the acquired reception location information and which is addressed to the user terminal 50c-2, and outputs the created location information to the communication unit 151.

### (Information Processing Device)

The information processing device 200c is implemented by a device such as a personal computer or an industrial computer. However, the information processing device 200c is not limited to the examples mentioned above. The information processing device 200c includes a communication unit 251, a memory 252, a storing unit 253c, an information processing unit 257c, and a bus line 263 such as an address bus or a data bus for electrically connecting the components of the information processing device 200c as illustrated in Fig. 18.

The communication unit 251 receives the location information acquisition request transmitted from the server 100c, and outputs the received location information acquisition request to the information processing unit 257c. The communication unit 251 acquires the location notification information output from the information processing unit 257c, and transmits the acquired location notification information to the server 100c.

The storing unit 253c is implemented by, for example, a RAM, a ROM, an HDD, a flash memory, a hybrid storage device including a combination of some of the RAM, the ROM, the HDD, and the flash memory, or the like. A program 254 and an application 255c that are to be executed by the information processing unit 257c are stored in the storing unit 253c. Furthermore, the user notification information 256 is stored in the storing unit 253c.

The application 255c causes the information processing device 200c to execute functions described below, in addition to functions similar to the functions that the application 255 causes the information processing device 200 to execute in the first embodiment. The application 255c causes the information processing device 200c to receive the location information notification transmitted from the server 100 and acquire the ID of the aerosol generation device 70 and the reception location information that are included in the received location information notification. The application 255c causes the information processing device 200c to create location information in which the acquired ID of the aerosol generation device 70 and reception location information are associated with each other and store the created location information.

The application 255c causes the information processing device 200c to receive the location information request transmitted from the server 100. The application 255c causes the information processing device 200c to acquire the ID of the aerosol generation device 70 included in the received location information request. The application 255c causes the information processing device 200c to acquire the reception location information stored in association with the ID of the aerosol generation device 70 from the stored location information, on the basis of the acquired ID of the aerosol generation device 70. The application 255c causes the information processing device 200c to create location notification information which includes the acquired reception location information and which is addressed to the server 100c and transmit the created location notification information to the server 100c.

The entire or part of the information processing unit 257c is a software functional unit that is implemented when, for example, a processor such as a CPU executes the program 254 and the application 255c that are stored in the storing unit 253c. The entire or part of the information processing unit 257c may be implemented by hardware such as an LSI, an ASIC, or an FPGA or may be implemented by a combination of the software functional unit and the hardware.

The information processing unit 257c includes, for example, a reception part 258 and a creation part 259c.

The reception part 258 acquires the location information notification output from the communication unit 251, and outputs the acquired location information notification to the creation part 259c. The reception part 258 acquires the location information acquisition request output from the communication unit 251, and outputs the acquired location information acquisition request to the creation part 259c.

The creation part 259c acquires the location information notification output from the reception part 258, and acquires the ID of the aerosol generation device 70 and the reception location information that are included in the acquired location information notification. The creation part 259c creates location information in which the acquired ID of the aerosol generation device 70 and reception location information are associated with each other, and stores the created location information into the storing unit 253c. The creation part 259c acquires the location information acquisition request output from the reception part 258, and acquires the ID of the aerosol generation device 70 included in the acquired location information acquisition request. The creation part 259c acquires the reception location information stored in association with the ID of the aerosol generation device 70 from the storing unit 253c, on the basis of the acquired ID of the aerosol generation device 70. The creation part 259c creates location notification information which includes the acquired reception location information and which is addressed to the server 100c, and outputs the created location notification information to the communication unit 251.

### (Operation of Information Provision System)

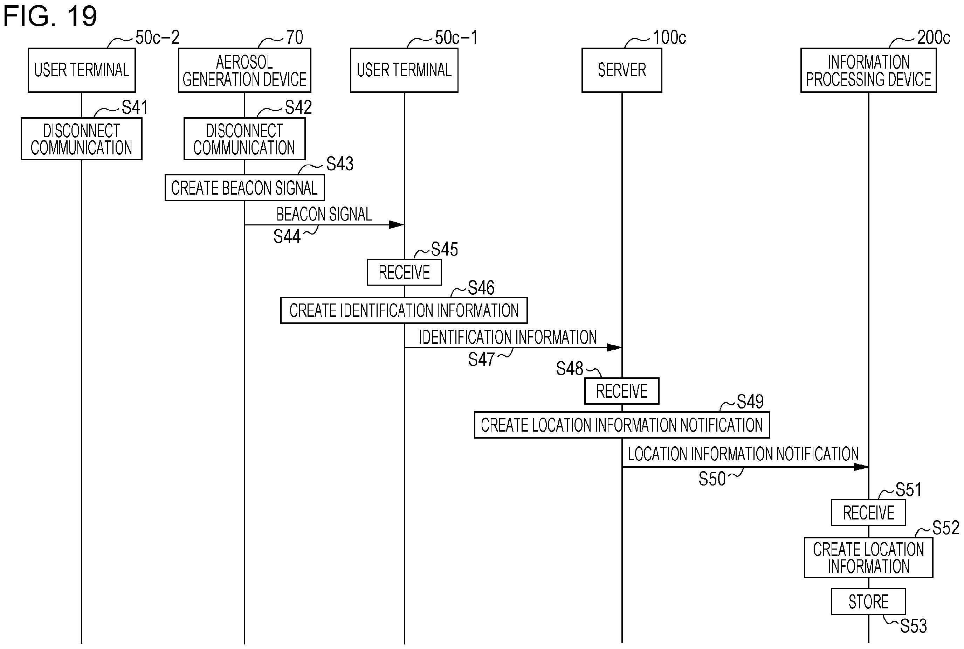

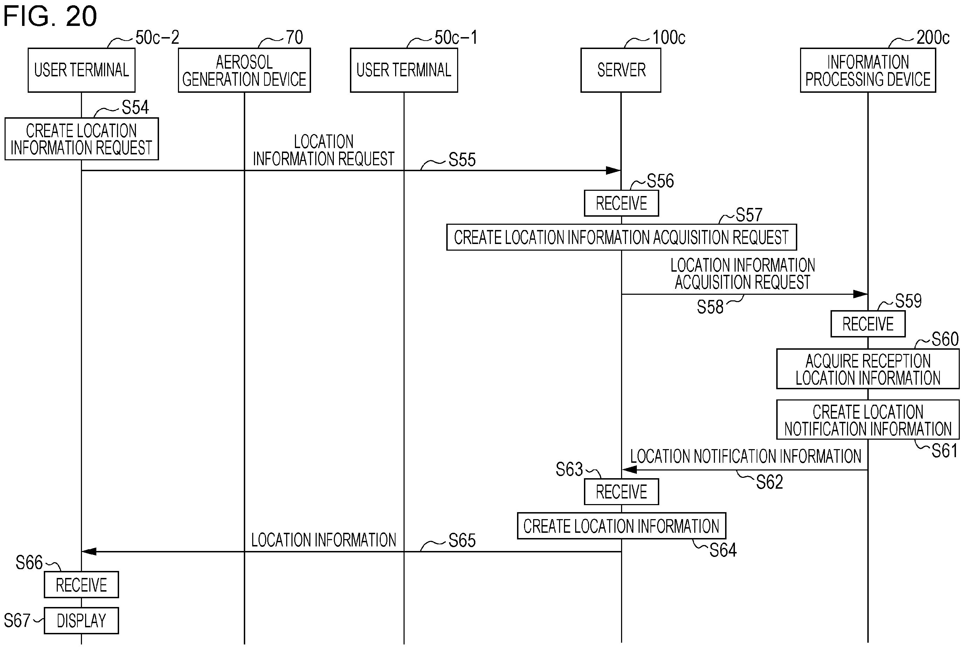

Figs. 19 and 20 are sequence charts illustrating an example of an operation of the information provision system according to the third embodiment. In the example illustrated in Figs. 19 and 20, a case where communication performed between the user terminal 50c-2 and the aerosol generation device 70 is disconnected will be described.

### (Step S41)

The communication unit 51 of the user terminal 50c-2 disconnects communication with the aerosol generation device 70.

### (Step S42)

The communication unit 71 of the aerosol generation device 70 disconnects communication with the user terminal 50c-2.

### (Step S43)

Disconnection of communication with the user terminal 50c-2 triggers the creation part 79 of the aerosol generation device 70 to create a beacon signal including a beacon ID and the ID of the aerosol generation device 70.

### (Step S44)

The creation part 79 of the aerosol generation device 70 outputs the created beacon signal to the communication unit 71. The communication unit 71 acquires the beacon signal output from the creation part 79, and transmits the acquired beacon signal.

### (Step S45)

The communication unit 51 of the user terminal 50c-1 receives the beacon signal transmitted from the aerosol generation device 70, and outputs the received beacon signal to the information processing unit 57.

Steps S26 to S31 described above with reference to Fig. 16 may be applied to step S46 to S51.

### (Step S52)

The creation part 259c of the information processing device 200c acquires a location information notification output from the reception part 258, and acquires the ID of the aerosol generation device 70 and reception location information that are included in the acquired location information notification. The creation part 259c creates location information in which the acquired ID of the aerosol generation device 70 and reception location information are associated with each other.

### (step S53)

The creation part 259c stores the created location information into the storing unit 253c.

Explanation will be continued below with reference to Fig. 20.

### (Step S54)

The reception part 58 of the user terminal 50c-2 acquires information output from the operation unit 61 indicating that the location information is requested, and receives the acquired information indicating that the location information is requested. The reception part 58 outputs the received information indicating that the location information is requested to the creation part 59c.

When the creation part 59c acquires the information output from the reception part 58 indicating that the location information is requested, the creation part 59c acquires the beacon ID and the ID of the aerosol generation device 70 from the storing unit 53c. The creation part 59c creates a location information request which includes the acquired ID of the aerosol generation device 70 and which is addressed to the server 100c associated with the beacon ID.

### (Step S55)

The creation part 59c outputs the created location information request to the communication unit 51. The communication unit 51 acquires the location information request output from the creation part 59c, and transmits the acquired location information request to the server 100c.

### (Step S56)

The communication unit 151 of the server 100c receives the location information request transmitted from the user terminal 50c-2, and outputs the received location information request to the information processing unit 157c.

### (Step S57)

The reception part 158 of the information processing unit 157c acquires the location information request output from the communication unit 151, and receives the acquired location information request. The reception part 158 outputs the received location information request to the creation part 159c. The creation part 159c acquires the location information request output from the reception part 158, and acquires the ID of the aerosol generation device 70 included in the acquired location information request. The creation part 159c creates a location information acquisition request which includes the acquired ID of the aerosol generation device 70 and which is addressed to the address of the information processing device 200c.

### (Step S58)

The creation part 159c outputs the created location information acquisition request to the communication unit 151. The communication unit 151 acquires the location information acquisition request output from the creation part 159c, and transmits the acquired location information acquisition request to the information processing device 200c.

### (Step S65)

The communication unit 251 of the information processing device 200c receives the location information acquisition request transmitted from the server 100c, and outputs the received location information acquisition request to the information processing unit 257c.

### (Step S60)

The reception part 258 of the information processing unit 257c acquires the location information acquisition request output from the communication unit 251, and outputs the acquired location information acquisition request to the creation part 259c. The creation part 259c acquires the location information acquisition request output from the reception part 258, and acquires the ID of the aerosol generation device 70 included in the acquired location information acquisition request.

### (Step S61)

The creation part 259c acquires the reception location information that is stored in association with the ID of the aerosol generation device 70 from the location information stored in the storing unit 253c, on the basis of the acquired ID of the aerosol generation device 70. The creation part 259c creates location notification information which includes the acquired reception location information and which is addressed to the address of the server 100c, and outputs the created location notification information to the communication unit 251.

### (Step S62)

The communication unit 251 acquires the location notification information output from the creation part 259c, and transmits the acquired location notification information to the server 100c.

### (Step S63)

The communication unit 151 of the server 100c receives the location notification information transmitted from the information processing device 200c, and outputs the received location notification information to the information processing unit 157c.

### (Step S64)

The reception part 158 of the information processing unit 157c acquires the location notification information output from the communication unit 151, and receives the acquired location notification information. The reception part 158 outputs the received location notification information to the creation part 159c. The creation part 159c acquires the location notification information output from the reception part 158, and acquires the reception location information included in the acquired location notification information. The creation part 159c creates location information which includes the acquired reception location information and which is addressed to the user terminal 50c-2, and outputs the created location information to the communication unit 151.

### (step S65).

The communication unit 151 acquires the location information output from the information processing unit 157c, and transmits the acquired location information to the user terminal 50c-2.

### (Step S66)

The communication unit 51 of the user terminal 50c-2 receives the location information transmitted from the server 100c, and outputs the received location information to the information processing unit 57c.

### (Step S67)

The reception part 58 of the information processing unit 57c acquires the location information output from the communication unit 51, and receives the acquired location information. The reception part 58 outputs the received location information to the processing part 60.

The processing part 60 acquires the location information output from the reception part 58, processes the acquired location information, and displays a result of the processing on the display unit 62.

With the information provision system 1c according to the third embodiment, the information processing device 200c creates location information in which the ID of the aerosol generation device 70 and the reception location information that are included in the location information notification transmitted from the server 100c are associated with each other, and stores the created location information. Thus, in the case where the user terminal 50c-2 transmits a location information request which includes the ID of the missing aerosol generation device 70 and which is addressed to the server 100c associated with the beacon ID, the information processing device 200c is able to provide, on the basis of the stored location information, the location information of the missing aerosol generation device 70 to the user terminal 50c-2.

In the first embodiment, the modification of the first embodiment, the second embodiment, and the third embodiment described above, the location information notification is an example of information relating to a beacon signal, the smoking area is an example of a place where an aerosol generation device can be used, and the reception part is an example of an acquisition unit.

The embodiments described above are presented as examples and are not intended to limit the scope of the present invention. The embodiments may be implemented in various other aspects. Various omissions, replacements, changes, and combinations may be made to the embodiments without departing from the gist of the present invention. The embodiments are included in the scope and gist of the present invention and included in the scope of the present invention described in the claims and equivalents thereof.

For example, the battery 710 described above may be configured to be a battery device 28. The battery device 28 is configured to be housed in the aerosol generation device 70.

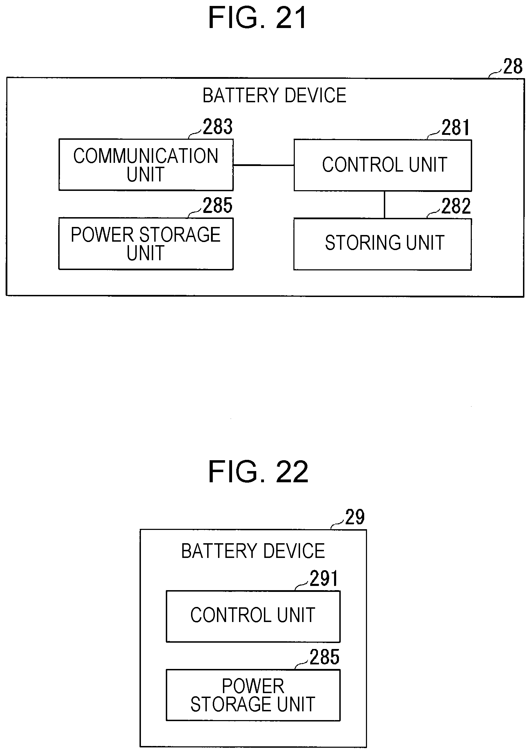

Fig. 21 is a block diagram illustrating a schematic functional configuration of a battery device according to an embodiment. As illustrated in the drawing, the battery device 28 includes a control unit 281, a storing unit 282, a communication unit 283, and a power storage unit 285.

The control unit 281 controls the entire battery device 28. In particular, the control unit 281 detects and obtains usage of the battery device 28 (status of connection with other devices, charging status of the battery device 28, etc.), writes and reads information to and from the storing unit 282, and transmits and receives information using the communication unit 283. The control unit 281 may be implemented by a computer and a program.

The control unit 281 is capable of reading a device ID for uniquely identifying the battery device 28 as an individual from the storing unit 282. The control unit 281 is capable of transmitting the device ID of the battery device 28 to the beacon transmission device 10 and the user terminal 50.

The storing unit 282 stores information for controlling the battery device 28. The storing unit 282 stores at least the device ID that is able to uniquely identify the battery device 28 as an individual. The storing unit 282 is implemented by, for example, a semiconductor memory.

The communication unit 283 includes a function for communicating with an external device using wireless communication means or the like. The communication unit 283 performs wireless communication, for example, within a short range, for example, which ranges from about several meters to about several hundred meters. The communication unit 283 implements communication between the battery device 28 and each of the beacon transmission device 10 and the user terminal 50.

The power storage unit 285 stores electric power. Electric power may be stored in the power storage unit 285 using a charging device (not illustrated in the drawing). Furthermore, electric power stored in the power storage unit 285 can be supplied to the aerosol generation device 70.

As described above, the battery device 28 includes the communication function. Thus, the beacon transmission device 10 and the user terminal 50 according to this embodiment are capable of directly receiving and processing various types of information from the battery device 28. For example, the battery device 28 includes the communication function and transmits a beacon signal to the beacon transmission device 10 and the user terminal 50.

Fig. 22 is a block diagram illustrating another schematic functional configuration of a battery device according to an embodiment. A battery device 29 includes a control unit 291 and a power storage unit 285. Unlike the battery device 28 illustrated in Fig. 21, the battery device 29 according to this embodiment does not include a function for directly communicating with the beacon transmission device 10, the user terminal 50, and the like. As described above, because the battery device 29 does not include a function of a communication unit or the like, a decrease in the cost of the battery device can be achieved.

The control unit 291 includes a function for controlling the entire battery device 29.

The power storage unit 285 stores electric power. Electric power may be stored in the power storage unit 285 by using a charging device (not illustrated in the drawing). Furthermore, electric power stored in the power storage unit 285 can be supplied to the aerosol generation device 70.

In this embodiment, the battery device 29 does not store a device ID for identifying the device as an individual. The battery device 29 does not directly communicate with the beacon transmission device 10 and the user terminal 50. The state in which the battery device 29 is connected to a charging device (not illustrated in the drawing) may be understood by the charging device (not illustrated in the drawing), regardless of whether the battery device 29 is housed or not housed in the aerosol generation device 70.

The beacon transmission device 10, the user terminal 50, the aerosol generation device 70, the server 100, the information processing device 200, the information processing device 200a, the user terminal 50c-1, the user terminal 50c-2, the server 100c, and the information processing device 200c described above may be implemented by computers. In this case, a program for implementing functions of functional blocks is recorded on a computer-readable recording medium. The functions may be implemented when the program recorded on the recording medium is read by a computer system and executed by a CPU. The "computer system" includes hardware such as an operating system (OS) and a peripheral device.

Furthermore, a "computer-readable recording medium" represents a portable medium such as a flexible disc, a magnetooptical disc, a ROM, or a CD-ROM. Furthermore, the "computer-readable recording medium" includes a storage device such as a hard disk built in a computer system.

Furthermore, the "computer-readable recording medium" may include a thing that dynamically holds a program for a short period of time. The thing that dynamically holds a program for a short period of time represents, for example, a communication line for the case where a program is transmitted via a communication link such as a network such as the Internet or a telephone line.

Furthermore, the "computer-readable recording medium" may include a thing that holds a program for a certain period of time, such as a volatile memory inside a computer system functioning as a server or a client. Furthermore, the above-mentioned program may implement part of the functions described above. Furthermore, the program mentioned above may be implemented by a combination with a program in which a function described above has already been recorded in the computer system. Furthermore, the program may be implemented using a programmable logic device. The programmable logic device is, for example, a field programmable gate array (FPGA).

### Reference Signs List

1, 1a, 1b ··· information provision system, 10 ··· beacon transmission device, 50, 50c-1, 50c-2, 50c ··· user terminal, 70 ··· aerosol generation device, 100, 100c ··· server, 200, 200a, 200c ··· information processing device, 11, 51, 71, 151, 251 ··· communication unit, 12, 52, 152, 252 ··· memory, 13, 53, 53c, 73, 153, 153c, 253, 253c ··· storing unit, 14, 54, 74, 154, 254 ··· program, 15, 55, 55c, 155, 155c, 255, 255a, 255c application, 17, 57, 57c, 77, 157, 157c, 257, 257c, 257a ··· information processing unit, 18, 58, 158, 258 ··· reception part, 19, 59, 59c, 79, 159, 159c, 259, 259a, 259c ··· creation part, 264 ··· derivation part, 60 ··· processing part, 61, 81 ··· operation unit, 62 ··· display unit, 21, 80 ··· detection unit, 63, 163, 263 ··· bus line, 156 ··· beacon related information, 256 ··· user notification information, 260 ··· point granting information, 261 ··· point information

## Claims

1. An information provision method comprising:
a step for acquiring information, relating to a beacon signal which is received by a user terminal associated with an aerosol generation device, from a server which is included in a message exchange system capable of transmitting a message to the user terminal;
a step for, on a basis of the information relating to the beacon signal that is acquired, creating provision information which pertains to the aerosol generation device and which is provided to a user of the aerosol generation device; and
a step for transmitting the provision information to the server.

2. The information provision method according to Claim 1, further comprising:
a step for, on a basis of the information relating to the beacon signal, deriving information indicating an acquisition status of the information relating to the beacon signal; and
a step for, on a basis of the information indicating the acquisition status, creating the provision information.

3. The information provision method according to Claim 2, wherein the acquisition status is a number of times that the user terminal has received the beacon signal during a predetermined period of time.

4. The information provision method according to Claim 2, wherein the acquisition status is a time during which the user terminal continues to receive the beacon signal.

5. The information provision method according to any one of Claims 2 to 4, further comprising:
a step for, on a basis of the information indicating the acquisition status, creating reward information, which indicates a reward to be granted to the user of the aerosol generation device, as the provision information; and
a step for transmitting the reward information to the server.

6. The information provision method according to any one of Claims 2 to 5, further comprising a step for transmitting the provision information to the server at a timing determined on a basis of the information indicating the acquisition status.

7. The information provision method according to any one of Claims 1 to 6, wherein the beacon signal is transmitted from a predetermined device provided in a place where the aerosol generation device is able to be used.

8. The information provision method according to any one of Claims 1 to 7, further comprising a step for acquiring the information, relating to the beacon signal which is transmitted on a basis of detection of the aerosol generation device, from the server.

9. The information provision method according to any one of Claims 1 to 7, further comprising a step for acquiring the information, relating to the beacon signal which is transmitted on a basis of detection of a predetermined signal transmitted from the aerosol generation device, from the server.

10. The information provision method according to any one of Claims 1 to 7, further comprising a step for acquiring the information, relating to the beacon signal which is transmitted from the aerosol generation device and received by another user terminal different from the user terminal, from the server.

11. The information provision method according to any one of Claims 1 to 7, further comprising a step for acquiring the information, relating to the beacon signal which is transmitted from the aerosol generation device in accordance with interruption of connection between the user terminal and the aerosol generation device, from the server.

12. The information provision method according to any one of Claims 1 to 7, further comprising a step for acquiring the information, relating to the beacon signal which includes location information of the aerosol generation device, from the server.

13. The information provision method according to Claim 12, further comprising a step for, on a basis of the location information, acquiring information, relating to the location information of the aerosol generation device, from the server, for the user of the aerosol generation device.

14. A program for causing a computer to execute:
a process for acquiring information, relating to a beacon signal which is received by a user terminal associated with an aerosol generation device, from a server which is included in a message exchange system capable of transmitting a message to the user terminal;
a process for, on a basis of the information relating to the beacon signal that is acquired, creating provision information which pertains to the aerosol generation device and which is provided to a user of the aerosol generation device; and
a process for transmitting the provision information to the server.

15. An information processing device comprising:
an acquisition unit that acquires information, relating to a beacon signal which is received by a user terminal associated with an aerosol generation device, from a server which is included in a message exchange system capable of transmitting a message to the user terminal;
a creation unit that creates, on a basis of the information relating to the beacon signal acquired by the acquisition unit, provision information which pertains to the aerosol generation device and which is provided to a user of the aerosol generation device; and
a communication unit that transmits the provision information to the server.

16. An information processing device comprising:
an acquisition unit that acquires information relating to a beacon signal which is received by a user terminal associated with an aerosol generation device;
a creation unit that creates, on a basis of the information relating to the beacon signal acquired by the acquisition unit, provision information which pertains to the aerosol generation device and which is provided to a user of the aerosol generation device; and
a communication unit that transmits the provision information to the user terminal.

17. A battery included in an aerosol generation device, the battery comprising:
a communication unit that communicates with a user terminal; and
a creation unit that creates a beacon signal in a case where connection with the user terminal is interrupted,
wherein the communication unit transmits the beacon signal created by the creation unit.

18. A terminal device associated with an aerosol generation device, the terminal device comprising:
a communication unit that receives a beacon signal; and
a creation unit that creates identification information which includes a beacon identifier that is able to uniquely identify the beacon signal which is included in the beacon signal that is received and a user identifier that is able to uniquely identify a user of the terminal device,
wherein the communication unit transmits the identification information to a server which is included in a message exchange system capable of transmitting a message to the terminal device.

19. An information provision system comprising:
an aerosol generation device that generates an aerosol;
a terminal device that is capable of communicating and connecting with the aerosol generation device;
a server that is included in a message exchange system capable of transmitting a message to the terminal device; and
an information processing device that receives information, relating to a beacon signal which is received by a user terminal associated with the aerosol generation device, from the server,
wherein the information processing device creates, on a basis of the information relating to the beacon signal that is acquired, provision information which pertains to the aerosol generation device and which is provided to a user of the aerosol generation device, and transmits the provision information that is created to the server.
